# EUROPEAN PATENT SPECIFICATION

(11) **EP 1 041 144 B1**
(45) Date of publication and mention of the grant of the patent: **23.06.2004**
(21) Application number: 99106453.6
(22) Date of filing: 29.03.1999
(51) Int. Cl.: C12N 15/10, C12Q 1/68

(54) **Methods for the identification and validation of functional intracellular targets with intramers or in vivo selection**
Methoden zur Identifizierung und Validierung von funktionellen Zielen mittels Intrameren oder in vivo Selektion
Procédé pour l'identification et la validation des cibles au moyen des intramers ou de la selection in vivo

(43) Date of publication of application: 04.10.2000
(73) Proprietor: Blind, Michael, 81245 München (DE); Famulok, Michael, 81245 München (DE); Kolanus, Waldemar, 81245 München (DE)
(72) Inventor: Blind, Michael, 81245 München (DE); Famulok, Michael, 81245 München (DE); Kolanus, Waldemar, 81245 München (DE)
(74) Representative: Bohmann, Armin K., Dr.

(56) References cited:
- WO-A-96/38553
- WO-A-97/27212
- WO-A-97/39722
- WO-A-98/32880
- US-A- 5 707 796
- BLIND ET AL.: "CYTOPLASMIC RNA MODULATORS OF AN INSIDE-OUT SIGNAL-TRANSDUCTION CASCADE" PNAS, vol. 96, March 1999 (1999-03), XP002113353
- KLUG ET AL.: "IN VITRO AND IN VIVO CHARACTERIZATION OF NOVEL mRNA MOTIFS THAT BIND SPECIAL ELONGATION FACTOR SelB" PNAS, vol. 94, 1997, pages 6676-6681, XP002113354
- FERBER AND MAHER: "COMBINATORIAL SELECTION OF A SMALL RNA THAT INDUCES AMPLIFICATION OF IncFII PLASMIDS IN ESCHERICHIA COLI" J.MOL.BIOL., vol. 279, June 1998 (1998-06), pages 565-576, XP002113817
- WERSTUCK AND GREEN: "CONTROLLING GENE EXPRESSION IN LIVING CELLS THROUGH SMALL MOLECULE-RNA INTERACTIONS" SCIENCE, vol. 282, 1998, pages 296-298, XP002113355
- FAMULOK AND JENNE: "OLIGONUCLEOTIDE LIBRARIES-VARIATIO DELECTAT" CURR.OPIN.CHEM.BIOL., vol. 2, 1998, pages 320-327, XP002113356
- BURKE J M ET AL: "IN VITRO SELECTION AND EVOLUTION OF RNA: APPLICATIONS FOR CATALYTIC RNA, MOLECULAR RECOGNITION, AND DRUG DISCOVERY" FASEB JOURNAL, vol. 7, no. 1, 1 January 1993 (1993-01-01), pages 106-112, XP000567892 ISSN: 0892-6638
- KOLANUS W ET AL: "ALPHALBETA2 INTEGRIN/LFA-1 BINDING TO ICAM-1 INDUCED BY CYTOHESIN -1, A CYTOPLASMIC REGULATORY MOLECULE" CELL, vol. 86, no. 2, 26 July 1996 (1996-07-26), pages 233-242, XP002040510 ISSN: 0092-8674

## Description

The present invention relates to a method for the identification of an intramer capable of binding to and modifying the function of a functional intracellular target and to the intracellular application of functional nucleic acids, termed "intramers", which affect the biological function of an intracellular component (e.g. inhibit its function within the context of a living cell by specifically complexing the component). It could be shown that a) intramers can be placed into a functional context inside cells regardless whether the target naturally binds nucleic acids or not; b) intramers can convey effects to intracellular sites in which a nucleic acid normally is not found. This method is useful to enable the elucidation of the biological role of a wide variety of intracellular components. The present invention furthermore relates to methods for the expression of randomized nucleic acid libraries inside cells to identify functional intramers which alter the phenotype of the cell in which they are expressed. This method is, e.g. useful for the identification of an intracellular target as functionally responsible for or involved in a certain cellular phenotype without any prior knowledge on the biological function of the component. Finally, the present invention relates to a T7-RNA expression cassette comprising a T7-promotor.

In recent years significant progress has been achieved in identifying the complete set of genetic information of various organisms. The genomes of procaryotes such as E.coli or eucaryotes such as S.cerevisiae (Goffeau et al., Science 274 (1996), 546-567) and C. elegans (The C. elegans Sequencing Consortium, Science 282 (1998), 2012-2018) were completely sequenced. It is estimated that the complete human genome will be known in 3-5 years. The challenge one has to face after these achievements, however, is to link a function to every single gene. For example, a major goal is to identify those gene products as potential drug targets, which play key roles in the complex network of protein interactions that ultimately lead to diseases (see e.g. Friedrich, Nat. Biotechnol. 14 (1996), 1234-7). To identify these key molecules among the 100000 human genes the number of possible candidate molecules is defined by comparing the protein composition of different developmental- or disease states of a cell by differential gene expression. The actual status of the protein composition of a cell can be obtained by analyzing the mRNA of the cell (for reviews of the various methods see Wan et al., Nat. Biotechnol. 14 (1996), 1685-1691). This method, however, only provides an indirect and not very accurate measure of the cell's current proteome status because the mRNA may long be degraded while the protein is still present, or a high amount of mRNA has been transcribed but cannot be translated for some reasons. It is also not possible to cover posttranslational modifications of the expressed proteins. An alternative way to cover the proteome-status of a cell via "proteomics" is 2D gel electrophoresis. Using this method one can attempt to cover the composition of all expressed proteins in a cell at a given time in the form of separate spots on a gel whereby each spot corresponds to an individual protein. By comparing different gels, different proteins or amounts of proteins can be identified. However, this method currently allows to identify at best about 20% of expressed proteins in a higher eucaryote. The detection of low copy number proteins is problematic. In addition, both methods only allow to look at the presence or absence of gene products in a particular developmental or disease state. They do not allow to state whether or not the identified differences in the expression pattern cause the cellular status or are just a consequence thereof.
The direct analysis of the function a certain protein complex, or one of its subunits or domains exhibits in a cellular process can be achieved via the genetic "knock-out" (homologous recombination, antisense technologies), or via overexpression or mutation of the protein. This, however, always results in an alteration of the genetic information of an organism which makes the interpretation of the results difficult. For example, the knock-out of a gene does not allow to draw conclusions which part or domain of a protein is important for its fuction. In addition, the expression of other genes is affected in most cases (for a review see: Proteome Research: New Frontiers in Functional Genomics,Springer-Verlag (1997), 1-30).

There is thus a high demand for specific intracellular inhibitors or modulators which can be applied in a certain time-window and which enable the analysis of a certain unaltered protein within its natural expression status [(Spencer et al., Science 262 (1993), 1019-1024; Huang & Schreiber, Proc. Natl. Acad. Sci. USA 94 (1997), 13396-13401]. Most inhibitors or modulators currently known are based upon membrane permeable small organic molecules which often are of limited specificity and are available only for particular proteins. This limitation may be overcome by intracellular antibodies ("intrabodies"; see Richardson, Tibtech 13 (1995), 306-10) or peptide aptamers (see Colas et al. Nature 380 (1996) 548-550). The problem with intracellular antibodies, however, is, for example, to adjust the extracellular antibody to the reductive intracellular compartment. Processes such as dimerization of heavy- and light chain, and the stabilization by disulfide bonds or glycosylation occur intracellularly with very low efficiency or not at all and have to be compensated by costly design of the polypeptide chains.

Functional nucleic acids acting within the context of a living cell can help to fulfill the demand for specific intracellular inhibitors or modulators which can be applied in a certain time-window and which enable the analysis of a certain unaltered protein within its natural expression status.

Functional nucleic acids are a single stranded DNA (ssDNA) or RNA (ssRNA) or chemically modified nucleic acids (ssNAmod), double stranded DNA (dsDNA) or RNA (dsRNA) or chemically modified forms thereof (dsNAmod) which are able to bind, modulate or catalytically modify an intracellular target thereby affecting its biological function. A functional nucleic acid can be identified for example through in vitro selection or "SELEX" (systematic evolution of ligands by exponential enrichment; see Tuerk and Gold, Science 249 (1990), 505-510; Ellington and Szostak, Nature 346 (1990) 818-822), or through ribozymes which can be activated allosterically (aptazymes; see Robertson and Ellington, Nat. Biotechnol. 17 (1999), 62-66). It is well established that functional nucleic acids, for example aptamers generated by in vitro selection, can bind to a wide variety of ligands ranging from small molecules and biological cofactors to natural and synthetic polymers including proteins, polysaccharides, glycoproteins, hormones, receptors and cell surfaces. In general, nucleic acids can be transferred into cells by methods commonly known to the expert such as lipofection, electroporation or by means of vectors which enable high rates of transcription, e.g. plasmids with RNA polymerase III promotors,T7 RNA polymerase/vaccinia virus based systems, or replication, e.g. Semliki forest virus.

Examples of small, intracellularly applied nucleic acids so far are limited to antisense nucleic acids, catalytic antisense nucleic acids such as hammerhead ribozymes (Birikh et al., Eur. J. Biochem. 245 (1997), 1-16; Bramlage et al., Trends Biotechnol. 16 (1998), 434-438) and nucleic acid ligands which bind via an aptameric mechanism to certain targets that are limited to naturally nucleic acid binding proteins, such as the Rev-protein from HIV-1 (Good et al., Gene Ther. 4 (1997), 45-54; Symensma et al., J. Virol. 70 (1996), 179-187), RNA polymerase II from yeast (Thomas et al., J. Biol. Chem. 272 (1997), 27980-27986), the SelB protein from E. coli (Klug et al., Proc. Natl. Acad. Sci. USA 94 (1997), 6676-6681). These examples, however, do not fulfill the demand for specific intracellular inhibitors or modulators which can routinely be applied in a certain time-window and which enable the analysis of a certain unaltered cellular component such as a protein within its natural expression status. So far it has only been established that in vitro selected aptamers which bind to a naturally nucleic acid-binding protein, and are usually closely related to the corresponding natural nucleic acid sequence, still do so inside the cell. It has not yet been established, for example, that an aptamer can generally be applied to affect the biological function of intracellular targets, including, for example, hormones, biological cofactors, non-nucleic acid-binding proteins, biopolymers etc.

International patent application PCT/DK96/00231 describes a method for the identification of biological active peptides and nucleic acids. The document discloses the basic steps such as production of a pool of appropriate vectors each containing totally or partly random DNA sequences, efficient transduction of said vectors into a number of identical eukaryotic cells, screening of said transduced cells to see whether some of them have changed a certain phenotypic trait, selecting and cloning of said changed cells, isolation and sequencing of the vector DNA in said phenotypically changed cells and deducing the RNA sequence from the DNA sequence. However, the respective vectors are not further characterised in terms of the expression cassettes used.

Thus, the technical problem underlying the present invention is to provide means for the identification and validation of intracellular targets including targets which naturally do not bind to nucleic acids and for the modification of the function of said target.

The solution to said technical problem is achieved by providing the embodiments characterized in the claims.

The present invention is based on the unique insight that nucleic acid ligands can be targeted against practically any cellular component, and can be easily applied intracellularly whereby
a) they still recognize their target in the intracellular environment;
b) they can do so even with targets that are non-nucleic acid-binding by nature;
c) they can localize their target in subcellular compartments such as the cytoplasmic face of the cell membrane where nucleic acids are normally not found;
d) they can modulate the biological function of the target which allows conclusions with respect to its biological role; and
e) they can alter the phenotype of the cell.

This enables the general application of intracellular functional nucleic acids for the validation of the function of intracellular targets, preferentially for the selection or validation of novel drug-targets. If desired, the functional nucleic acid sequence can be placed within an expression cassette or other sequence context that may be useful for
a) increasing the stability of the functional nucleic acid within the cellular compartment or providing signals, e.g. for correct termination of a functional RNA sequence that is expressed under the control of RNA polymerases; and
b) providing additional sequence information that is required for the correct localization of the functional nucleic acid, e.g. to transfer the nucleic acid from the nucleus to the cytoplasm.

Once brought into the cell, these nucleic acids will excert their biological effects which can be studied. The method thus could significantly contribute to the field of "functional genomics".

Accordingly, in a first aspect the present invention relates to a method for the identification of an intramer capable of binding to and modifying the function of a functional intracellular target, comprising:
a) preparing a candidate mixture of nucleic acids;
b) contacting the candidate mixture of nucleic acids with the intracellular target or a part thereof;
c) selecting and isolating nucleic acids having an increased affinity to said target relative to the candidate mixture;
d) reverse transcribing, if the candidate mixture comprises RNAs and amplifying the nucleic acids obtained in step c);
e) optionally repeating steps b) to d);
f) isolating and sequencing the clones (intramers) obtained in step e); and
g) testing whether the expression product of the insert of the clone obtained in step f) binds to and effects the function of the intracellular target in vivo wherein a T7-RNA expression cassette is used as a cytoplasma expression system which comprises a T7 promoter, a stabilising 5' stem-loop and a 3' terminator Tφ with the sequence of said T7-RNA expression cassette with no insert being as follows: 
5'-GTTAAC-GCATGCTAATACGACTCACTATAG
GGAGACCACAACGGTTTCCCGGGCGCAAGTTACTAGT-TGGCCA-
AGATCT-TAATTAATAGCATAACCCCTTGGGGCCTCTAAACGGG
TCTTGAGGGGTTTTTTGCTGTCGAC-GCGGCCGC-3'
 whereby the insert is inserted between the 5' stem-loop and the terminator Tφ via the XmaI and PacI restriction sites.
   Preferably, step g) is carried out by using a cytoplasmic expression system.
   In a more preferred embodiment the method of the present invention furthermore comprises
h) mapping of the binding site of the aptamer to said target.

The present invention furthermore relates to methods for the expression of randomized nucleic acid libraries inside cells for the identification of functional intramers which alter the phenotype of the cell in which they are expressed. Based on the inventor's insight of the general applicability of functional nucleic acids to affect intracellular targets it is also feasible to express libraries of randomized RNA sequences inside cells. These complex libraries of randomized nucleic acids contain functional molecules which bind to cellular components such as enhancers or repressors of signal transduction pathways and modulate their biological function resulting in an altered phenotype of the cell. These molecules can be identified with appropriate selection methods. Modulation not necessarily involves mechanisms that rely on mere binding but can also, for example, involve catalytic processes, such as ribozymatic modification of an intracellular target.

Once a certain phenotype is observed, it is possible to identify the cellular target(s) responsible for the phenotypic change. This can be done by variations of different technologies such as the "three hybrid system (SenGupta et al., Proc. Natl. Acad. Sci. USA, 93 (1996), 8496-8501) or other methods described in the examples below. In general, it is possible to identify the cellular target by taking advantage of the interaction between the active RNA from the library with the target. This system is useful for the targeted identification of key molecules involved in a certain phenotype, preferentially a phenotype associated with a certain disease. It is superior to other systems currently available because it circumvents the need for screening a large number of potential candidates which have been identified indirectly, for example by proteomics, but rather identifies the key molecules in a direct phenotypic selection process. The identified key molecules are potential drug targets; the nucleic acid modulators are not only target validators, but also potential drug-leads which could even be applied as drugs directly, for example in gene therapeutic approaches.

Accordingly, in a further aspect the present invention relates to a method for the identification of a functional intracellular target which is associated with a particular phenotype and the corresponding intramer capable of binding to and modifying the function of said target, comprising:
a) preparing a candidate mixture of nucleic acids;
b) cloning the candidate mixture of nucleic acids under the control of a suitable promotor in a vector optionally containing a selectable marker wherein the vector is a cytoplasmic expression system comprising a T7-RNA expression cassette wherein said T7-RNA expression cassette comprises a T7 promotor, a stabilising 5' stem-loop and a 3' terminator Tφ wherein the sequence of the T7-RNA expression cassette with no insert being as follows: 
5′-GTTAAC-GCATGCTAATACGACTCACTATAG
GGAGACCACAACGGTTTCCCGGGCGCAAGTTACTAGT-TGGCCA-
AGATCT-TAATTAATAGCATAACCCCTTGGGGCCTCTAAACGGG
TCTTGAGGGGTTTTTTGCTGTCGAC-GCGGCCGC-3′
 whereby the insert is inserted between the 5' stem-loop and the terminator Tφ via the XmaI and PacI restriction sites.
c) introducing the vector obtained in step b) into a reporter cell line allowing positive or negative phenotypic selection;
d) selecting cells with an altered phenotype; and
e) determining the sequence of the nucleic acid inserted in the vector of step b) (intramer) and the compound which it binds to.

### Brief description of the Figures

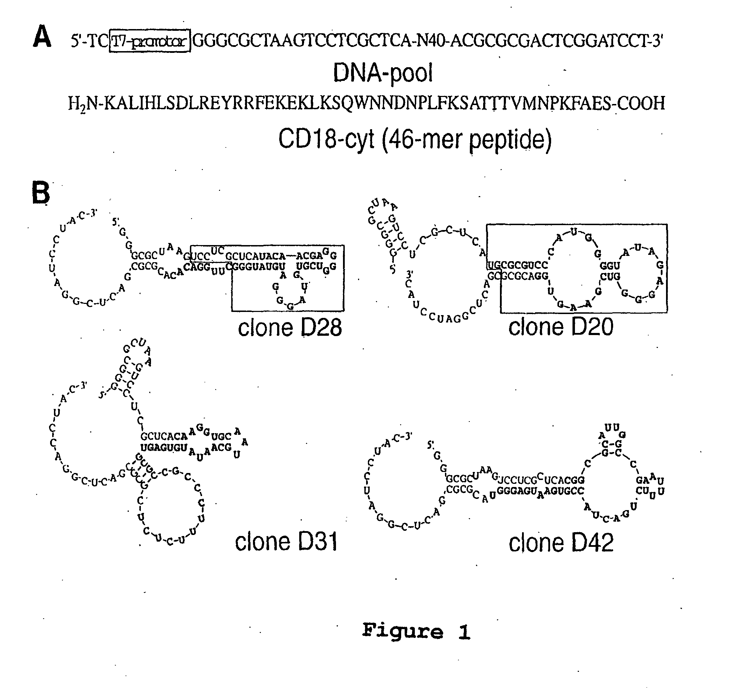

### A: Consecutive outline of Figures 1 - 5 (for Example 1)

Figure 1: Selection and characterisation of CD18cyt-specific RNA aptamers. **A**, Construction of the synthetic DNA pool and primary 46-mer amino acid sequence of the complete cytoplasmic domain of β2-integrin (CD18cyt), immobilised to sepharose and used in the selection. **B**, Sequences and predicted secondary structures of individual aptamer clones D20, D28, D31, and D42. Nucleotides shown in bold-face are part of the randomised region. The aptamer region shaded in grey in clones D28 and D20 were shown in a damage selection experiment to be minimal requirements for retaining CD18cyt-binding capacity. The sequence D42 was used as a negative control; this sequence does not show any detectable binding to CD18cyt.

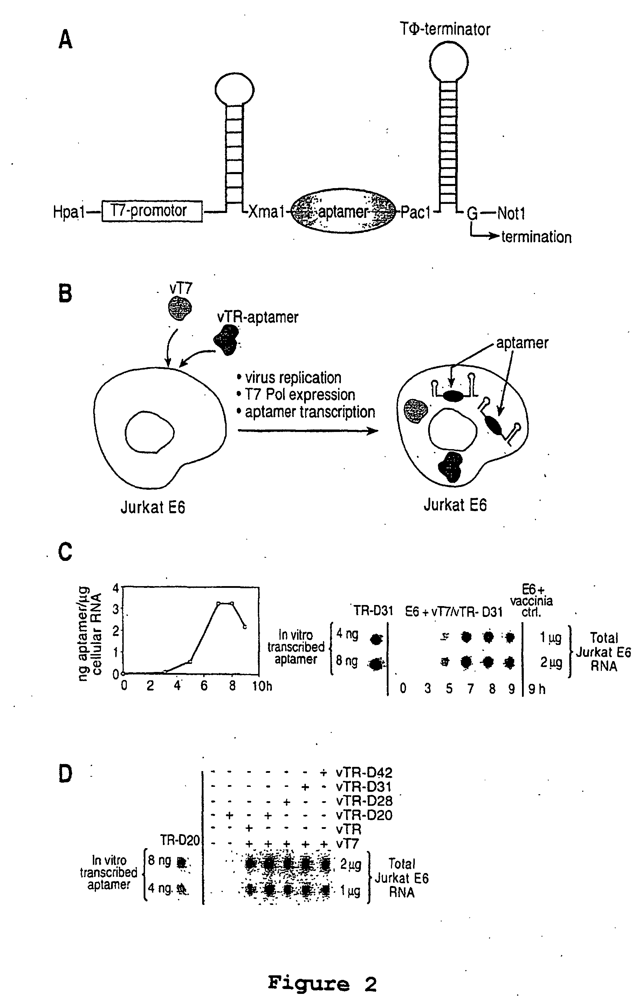

Figure 2: RNA aptamer expression system based on double infection with recombinant vaccinia viruses. **A**, Design of the T7-RNA expression cassette. **B**, Schematic representation of the vaccinia virus-based cytoplasmatic RNA aptamer expression system. **C**, Course of the expression of TR-encoded aptamer in vT7 coinfected Jurkat E6 cells shown by a representative dot blot analysis for aptamer TR-D31 (right panel). Maximum levels of aptamer expression are seen 7 h post infection. Quantification (left panel) was done as follows: total cellular RNA was isolated, transferred to the blotting membrane and hybridized with 5'-³²P-labeled oligonucleotide complementary to the 3'- stem-loop structure present in all TR-constructs. For comparison and quantification *in vitro* transcribed aptamer TR-D20 was treated in the same way. **D**, Dot blot analysis and quantification of maximally expressed aptamer RNA. Each dot was quantified on a phosphor imager. Quantification was done as described for Figure 2c.

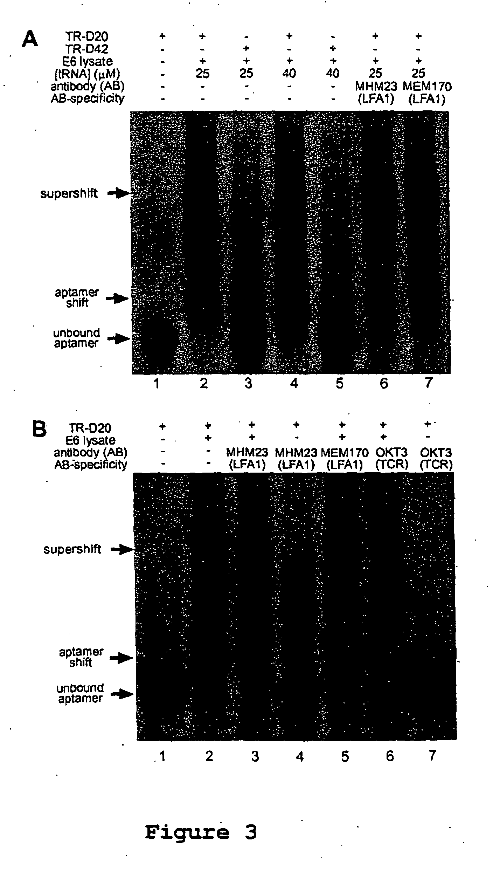

Figure 3: Gel mobility shift assays of endogenous CD18 contained in Jurkat E6 cell lysates bound to its cognate aptamer TR-D20. **A**. Gel shift experiment 1. Lane 1: free TR-D20 aptamer RNA; lane 2: shifted band obtained in presence of Jurkat E6 lysate and 25 µM unspecific competitor tRNA; lane 3: no shift obtained with negative control sequence TR-D42; lane 4: same as lane 2 with 40 µM unspecific competitor tRNA; lane 5: same as lane 3 with 40 µM unspecific competitor tRNA; lane 6: specific supershifted band obtained in the presence of antibody MHM23 which recognizes the β2-extracellular domain of LFA-1; lane 7: specific supershifted band obtained in the presence of antibody MEM170 which recognizes the extracellular domain of the βL-subunit of LFA-1. The band pattern may reflect aptamer/integrin complexes of different stoichiometry. **B**. Gel shift experiment 2 with additional controls. All gel shift experiments were performed in the presence of 30 µM tRNA as a non-specific competitor. Lane 1: free TR-D20 aptamer RNA; lane 2: shifted band obtained in presence of Jurkat E6 lysate; lane 3: specific supershifted band obtained in the presence of antibody MHM23; lane 4: no shifted aptamer obtained in the absence of Jurkat E6 cell lysate under conditions identical to those in lane 3. lane 5: specific supershifted band obtained in the presence of antibody MEM170; lane 6: no supershift obtained in the presence of antibody OKT3 directed against the T-cell receptor. The experimental conditions are otherwise the same as in lane 2; lane 7: no shifted aptamer obtained in the absence of Jurkat E6 cell lysate under conditions identical to those in lane 6.

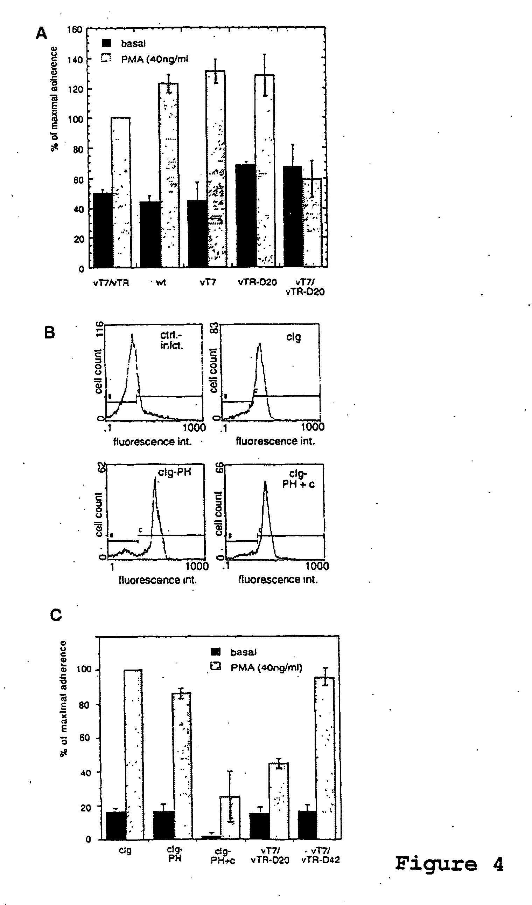

Figure 4: Inhibition of PMA-stimulated cell adhesion as a function of aptamer expression. **A**. The CD18cyt binder TR-D20 reduces phorbol-ester activated Jurkat cell adhesion to ICAM-1. Jurkat E6 cells infected with recombinant vaccinia viruses were allowed to adhere to plastic dishes coated with a recombinant ICAM-1 chimera as described in example 1 section. Jurkat E6 cell adhesion to ICAM-1 was superinducible by PMA in the presence of several control viruses (vT7/vTR, wild-type vaccinia virus, vT7, vTR-D20). However, induction of CD18cyt-specific aptamer expression (vT7/vTR-D20, right panel) reduced PMA-stimulated Jurkat E6 cell adhesion, but not basal adhesion. These results were independently reproduced at least three times. Percentage reflects values normalized to stimulated vT7/vTR double infection which was set to 100%. **B**. Human peripheral blood mononuclear cells are good targets for protein overexpression by recombinant vaccinia viruses. Cytoplasmic immunoglobulin (cIg) fusions of cytohesin-1 subdomains were detected with the help of a FITC-conjugated anti-human Ig antibody preparation in permeabilised PBMC. Upper panel, left: control infection, no recombinant molecules expressed ; upper panel, right: cIg-domains alone; lower panel, left: cIg-PH; lower panel, right: cIg-PH+c-domain. **C**. TR-D20 specifically reduces PMA-stimulated adhesion of PBMC to ICAM-1. Aptamers or control proteins were expressed in PBMC by recombinant vaccinia viruses as described above. PMA-stimulated adhesion of these cells to ICAM-1 was equivalent when the TR-D42 aptamer, cIg, or cIg-PH control proteins were expressed, but stimulated cell adhesion was dramatically reduced following expression of the intact PH + c-domain of cytohesin-1 (cIg-PH+c) or of the TR-D20 aptamer.

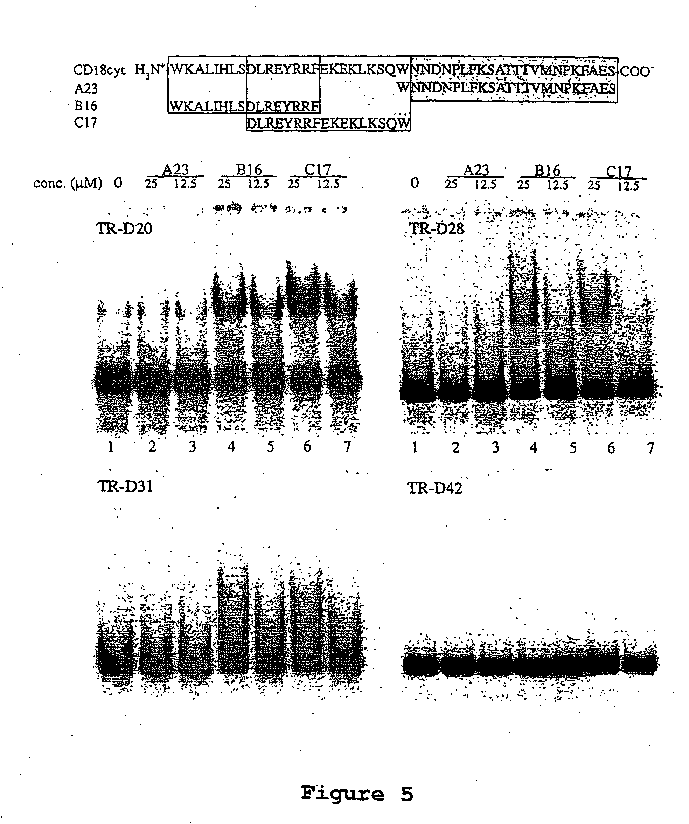

Figure 5: Mapping of the binding site of aptamers TR-D20, TR-D28, TR-D31, and the negative control sequence TR-D42 on CD18cyt using synthetic biotinylated peptide fragments. All gel shifts were carried out in presence of 25 µM streptavidine to enhance separation of shifted- versus non-shifted RNA. Gel shift experiments were performed in presence of 4 nM radiolabeled RNA and 40 µM nonspecific tRNA competitor. Lane 1: free aptamer in the presence of 25 µM streptavidine and 40 µM unspecific competitor tRNA; lanes 2, 4, 6 or 3, 5, 7: same as lane 1 in presence of 25 µM or 12.5 µM, respectively, peptides A23 (lane 2, 3), B16 (lane 4, 5), and C17 (lane 6, 7).

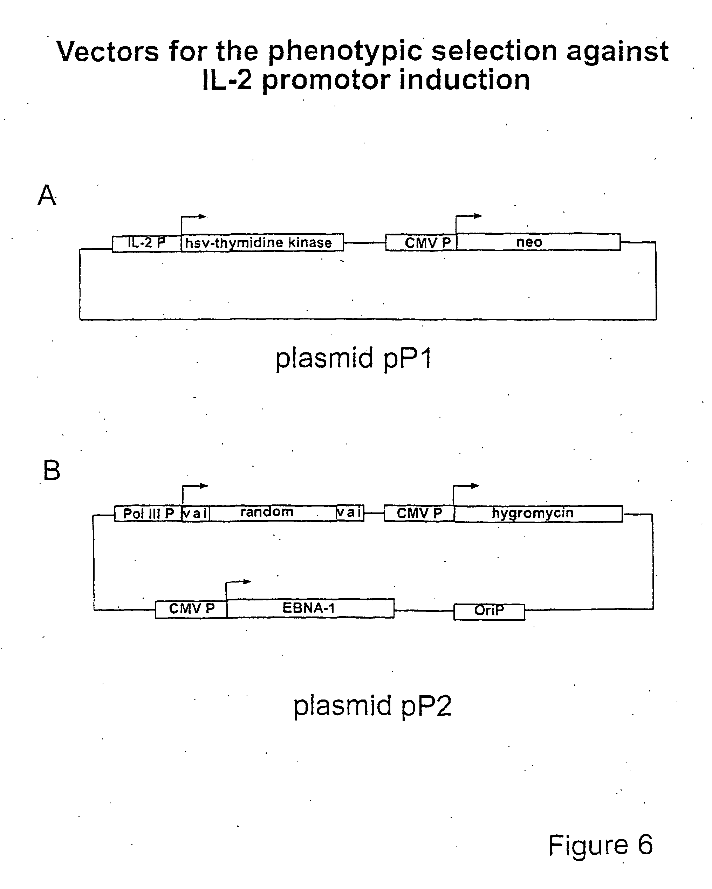

### B: Consecutive outline of Figures 6 - 12 (for Example 2)

Figure 6: Vectors for the phenotypic selection. **A** Scheme of important features of plasmid pP1; *CMV P*: promotor element: human cytomegalovirus immediate early promotor. Example of a constitutive promotor element that is active in a wide variety of cell types. Depending on cell lines, application, etc. other frequently used promotors or combinations of promoter elements (e.g. SV 40 early promotor, Rous sarcoma virus immediate early promotor etc.) can be used; *IL-2 P:* IL-2 promotor; *neo:* Selectable marker: commonly used resistance gene (aminoglycosid phosphotransferase) to select for cells harbouring the transfected plasmids via detoxification of aminoglycosid antibiotics; *hsv thymidine kinase:* gene for the herpes simplex virus thymidine kinase. **B** Scheme of important features of plasmid pP2; *CMV P*: see pP1; *Pol III P:* promotor element: RNA Polymerase III promotor; *vai random vai:* random nucleic acid library with the adenoviral VAI RNA sequences that permit the export of the functional nucleic acid into the cytoplasm; *OriP:* Episomal replication: The Epstein Barr virus (EBV) origin of replication and nuclear antigen (EBNA-1) allow the high copy episomal (extra chromosomal) replication and maintenance in primate and canine cell lines; *EBNA-1:* gene for the Ebstein Barr virus nuclear antigen1; *hygromycin:* Resistance against hygromycin can be used to co-select for cells that harbor two plasmids (e.g. an additional plasmid with the neo resistance)

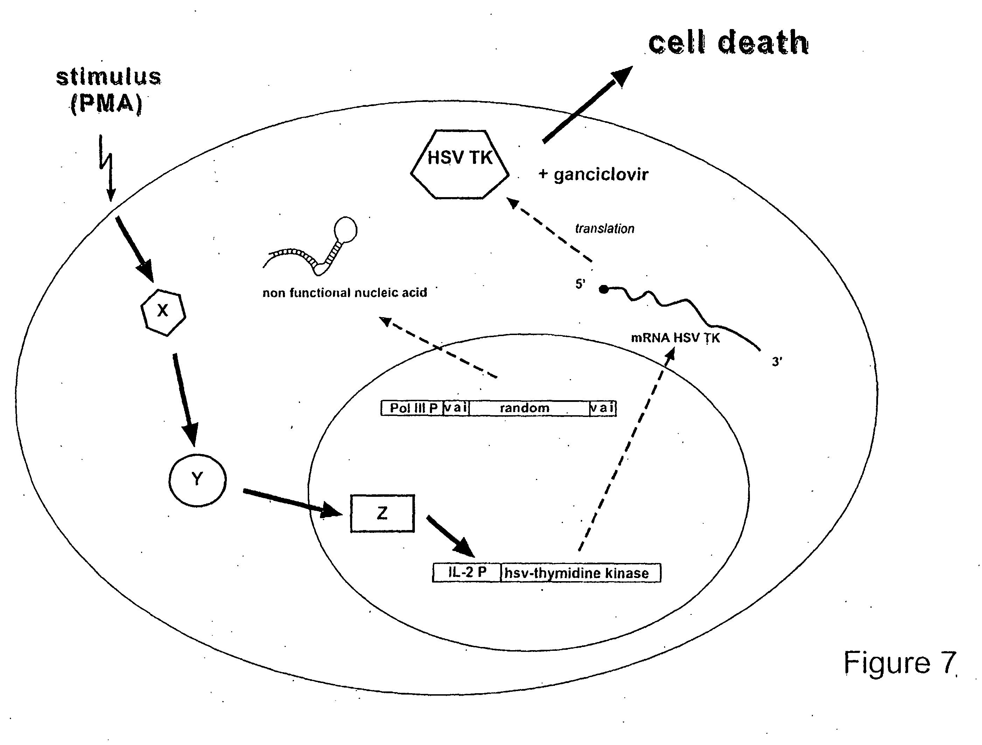

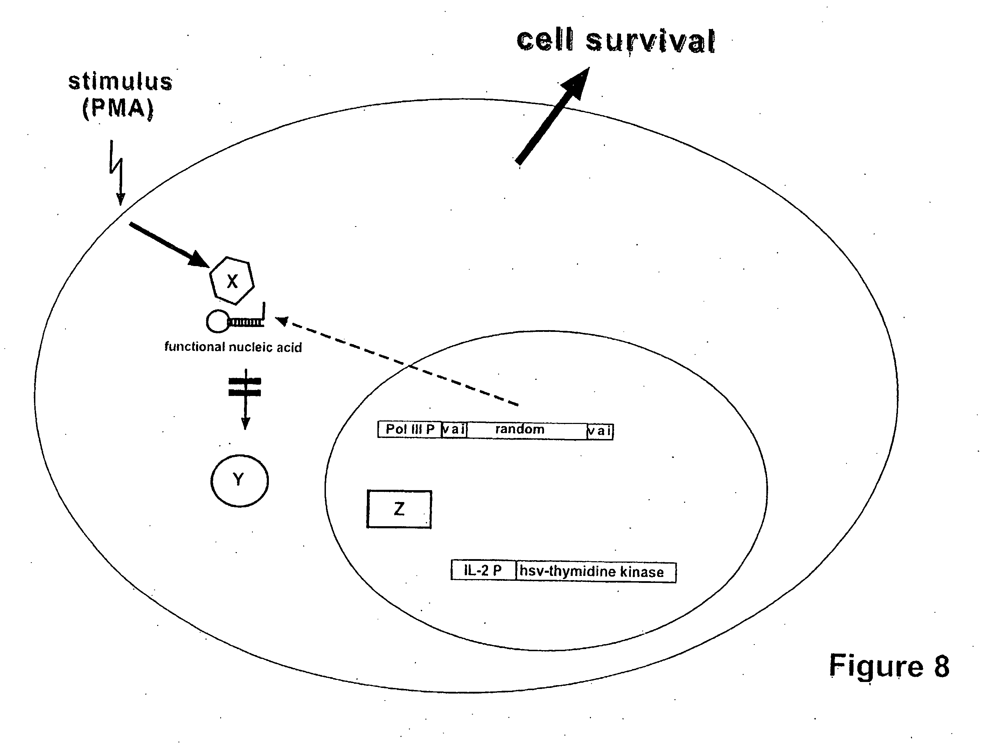

Figure 7: Scheme for the functional selection against the induction of the interleukin 2 promotor in T-cells after stimulation with phorbol-12-myristate-13-acetate (PMA). The protein cascade which is shown schematically in this example leading from protein X via Y to Z (straight arrows) in a T-cell, ultimately results in the activation of the interleukin-2 promotor (IL-2P) of the reporter construct IL-2 P/hsv-thymidine kinase. Transcription and translation of the mRNA of hsv-thymidine kinase (mRNA HSV TK) results in the expression of Herpes simplex thymidine kinase (HSV TK) (dashed arrows represent gene expression). Constitutive transcription of the sequences of a nucleic acid library (vai/random/vai) under the control of an RNA polymerase III promotor (Pol III P) results in the formation of a randomized RNA library which is exported into the cytoplasm due to its adenoviral VAI sequence. As indicated in this figure, the expression of a non functional nucleic acid does not affect the induction of IL-2P by the signal transduction cascade X, Y, Z, and the expression of the HSV TK. The presence of the HSV TK and ganciclovir will lead to T-cell death due to the production of toxic compounds. No cell death will occur if the expressed RNA library contains functional sequences which affect the expression of HSV TK, for example by interrupting the signal transduction cascade by inhibiting one of its proteins. These cells can then be selected (see Fig. 8).

Figure 8: Same phenotypic selection scheme as in Figure 7 but in this case a functional nucleic acid is expressed from Pol III/vai/random/vai (dashed arrows represent gene expression). The functional nucleic acid in this example binds to protein X and blocks the signalling from X to Y in the cascade. (straight arrows). Therefore, the induction of the IL-2 promotor is inhibited and no HSV TK expressed. The T cell survives in the presence of ganciclovir and the gene coding for the functional nucleic acid can be isolated.

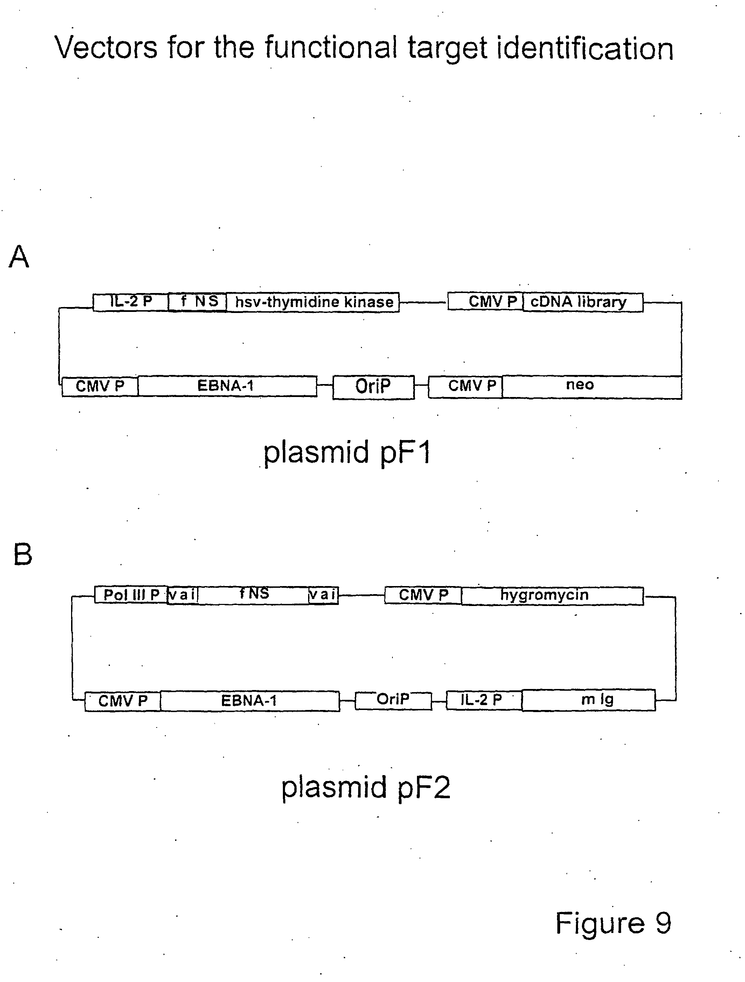

Figure 9: Vectors for the functional target identification. **A** Scheme of important features of plasmid pF1; *CMV P*: promotor element "human cytomegalovirus immediate early promotor". Example of a constitutive promotor element that is active in a wide variety of cell types; *IL-2 P:* IL-2 promotor; *OriP:* Episomal replication. The Epstein Barr Virus (EBV) origin of replication and nuclear antigen (EBNA-1) allow the high copy episomal (extra chromosomal) replication and maintenance in primate and canine cell lines; *EBNA-1:* gene for the Ebstein Barr virus nuclear antigen 1; *cDNA library:* The cDNA library in this example is derived from T cells. The library is used to select for the complementation of the inhibited signal transduction cascade. Overexpression of the factor that is inhibited by the functional nucleic acid bypasses the block in signalling in which case the IL-2 promotor is induced again in those cells which harbour the target gene of interest; *fNS-hsv thymidine kinase:* this construct allows the identification of the target gene product through the specificity of the nucleic acid-protein interaction. The gene of the functional nucleic acid (f NS), in this example an aptamer sequence, is cloned in the 5'-UTR of the herpes simplex virus thymidine kinase. Upon binding of the aptamer ligand (the functional nucleic acid) the expression of the reporter HSV-TK is inhibited presumably through blocking the translation; *neo:* Selectable marker and commonly used resistance gene (aminoglycosid phosphotransferase) to select for cells harbouring the transfected plasmids via detoxification of aminoglycosid antibiotics. **B** Scheme of important features of plasmid pF2. *CMV P*: see plasmid pF1; *Pol III P:* promotor element: RNA Polymerase III promotor; *vai fNS vai:* functional nucleic acid (fNS) with the adenoviral VAI RNA sequences that permit the export of the functional nucleic acid into the cytoplasm; *OriP:* See pF1; *hygromycin:* Resistance against hygromycin can be used to co-select for cells that harbor two plasmids (e.g. an additional plasmid with the neo resistance); *mIg:* Surface marker that allows the affinity selection of transformed cells. For example, in this example the constant CH2 and CH3 domains of human IgGl are expressed as a transmembrane version (m Ig) which is presented on the cell surface.

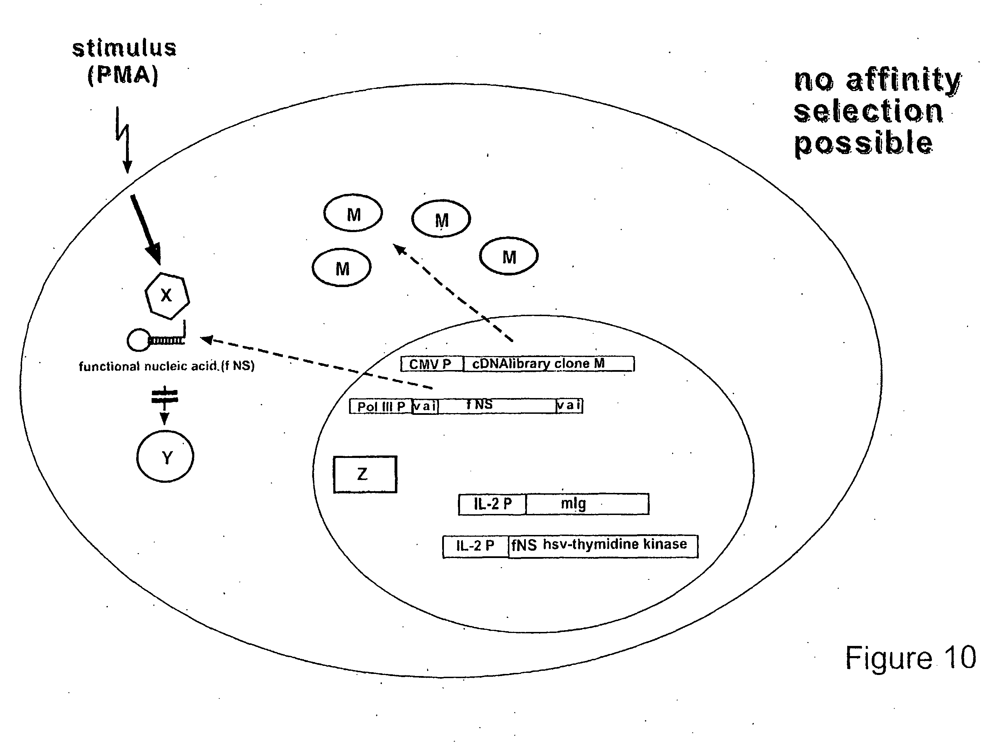

Figure 10: Scheme for the functional target identification. The functional nucleic acid identified in the phenotypic selection expressed from Pol III/vai/fNS/vai (dashed arrows represent gene expression) binds to protein X and blocks the signalling from X to Y (straight arrows) in the cascade after stimulation with phorbol-12-myristate-13-acetate (PMA) (see also Fig.8). As protein M (clone of a T cell cDNA library) which is expressed under the control of the promotor CMV P (see description of Fig. 9) has no effect on the blocked cascade, the induction of the IL-2 promotor (IL-2 P) is inhibited and hence the expression of the reporter construts fNS hsv-thymidine kinase and mIg (see description of Fig. 9) does not occur. As the T cells do not express the mIg on their surface they can not be isolated using for example antibodies against mIg.

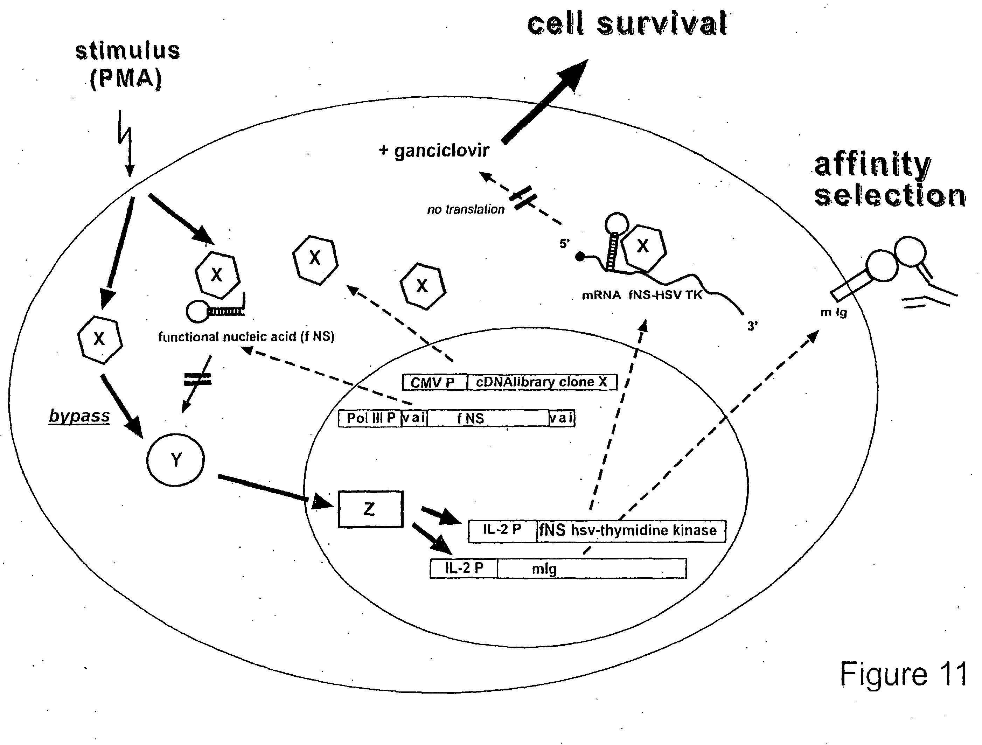

Figure 11: Scheme for the functional target identification. Same as Fig. 10, except that in this example the cognate target for the functional nucleic acid (fNS) protein X is expressed from the cDNA libray (dashed arrows represent gene expression). The overexpression of protein X bypasses the block of the endogenous protein X by the fNS. Therfore, after stimulation with PMA, the signal transduction cascade X,Y,Z (straight arrows) is rescued and the mRNA is transcribed from IL-2 promotor reporter constructs. Expression of the surface mIg (see also Fig.9) allows the affinity isolation of the T cells. Simultanously protein X binds to the fNS inserted in the 5`UTR of the fNS HSV TK construct. This results in the block of the translation of the HSV TK. The absence of the enzyme herpes simplex thymidine kinase enables the T cells to survive in the presence of ganciclovir and the positive cDNA clones can be isolated.

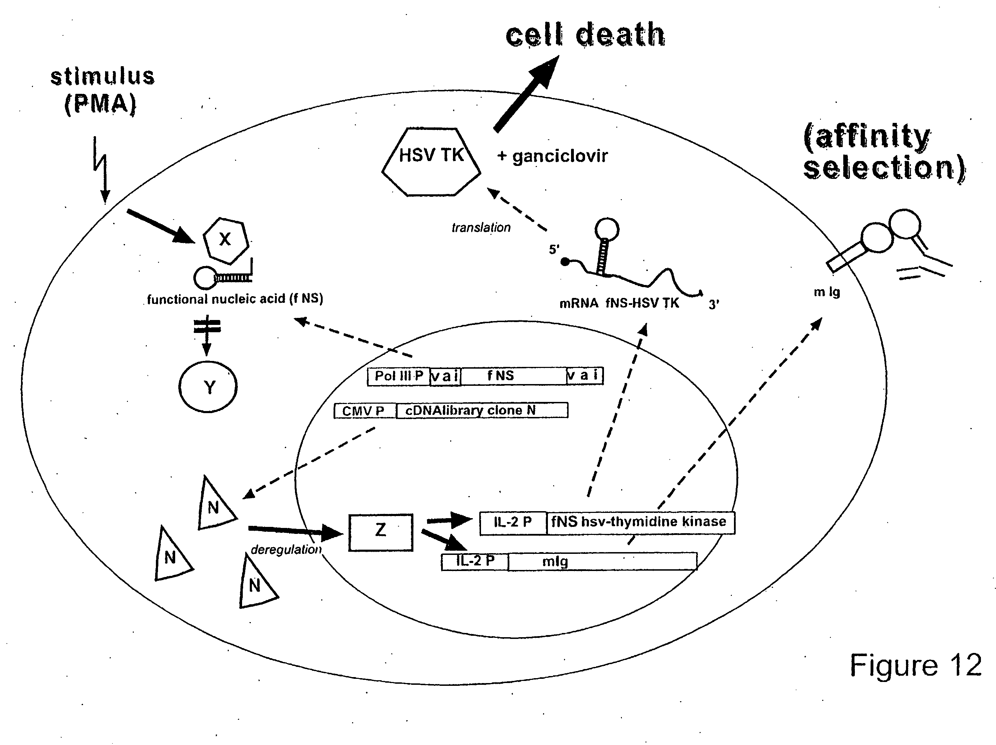

Figure 12. Scheme of the functional target identification. Although protein X is blocked by the functional nucleic acid (fNS) in this schematic example the protein N expressed from the cDNA library induces the I1-2 promotor reporter constructs by deregulating the endogenous signal transduction cascade by acting directly on protein Z (see also Figures 10, 11). This false positive complementation leads to the expression of the mIg which would allow the isolation of the cells with mIg specific antibodies. As protein N is not the cognate ligand for the functional nucleic acid it fails to bind to the fNS in the fNS-HSV TK mRNA construct (compare also Figures 10, 11). Therefore the herpes simplex thymidine kinase is expressed and the cells are killed in the presence of ganciclovir. This step eliminates false positive cDNA clones which act by deregulating the signal transduction.

The candidate RNAs or DNAs comprise functional nucleic acids i.e. a single stranded DNA (ssDNA) or RNA (ssRNA) or chemically modified forms thereof (ssNAmod), double stranded DNA (dsDNA) or RNA (dsRNA) or chemically modified forms thereof (dsNAmod) which are able to bind, modulate or catalytically modify an intracellular target thereby affecting its biological function. The functional nucleic acids can be identified, for example, by screening large combinatorial nucleic acid libraries (in vitro selection of ribozymes, aptazymes, aptamers etc.). Combinatorial nucleic acid libraries whether DNA or RNA usually contain a randomized sequence flanked by fixed regions which serve as primer annealing sites for enzymatic amplification via PCR or in the case of RNA via reverse transcription and PCR. The libraries can for example be sythezised as ssDNA on an automated DNA syntheziser using 3`phosphoramidite chemistry, wherby the randomized region is synthezised with 3' phosphoramidite solutions that contain mixtures of the four bases A,C,G,T (Hermes et al., Proc. Natl. Acad.Sci. U.S.A.87 (1990), 696-700). The ratios between the four monomers can be varied depending on the application. For preparation of RNA the fixed regions contain a RNA polymerase promotor, e.g. T7 RNA polymerase promotor, which allows the transcription of the template DNA into RNA. If necessary the ssDNA can be made doublestranded using enzymatic protocolls, e.g. PCR, which are well known to a person skilled in the art. For an overview see Abelson, Methods in Enzymology 267 (1996), pp 275-426, Academic Press, San Diego. Preferentially the library contains 8 to 200 randomized nucleotides (Tuerk and Gold, Science 249 (1990), 505-510; Bartel and Szostak, Science 261 (1993), 1411-1418) with complexities from 6 x 10⁵ to 1 x 10¹⁶ which have been used in successful selection experiments. If desired, the functional nucleic acid can be covalently attached or non convalently bound to other molecules such as chemically reactive groups, lipids, peptides, proteins etc. which add additional properties to the functional nucleic acid. Functional nucleic acids do not include nucleic acids acting by nucleic acid interactions such as antisense mechanisms or natural ribozymes or modified and mutated forms thereof which combine antisense recognition with phosphodiester cleavage activity and are applied, for example, in gene therapeutic approaches. A functional nucleic acid can be part of a construct with additional sequences whether natural or non natural which add further functions, e.g. enhanced stability, localisation signals or enzymatic activities. Furthermore, upon binding its intracellular target the functional nucleic acid itself can be modulated in a second function whether intrinsic or added in a construct with additional sequences for example binding a third ligand or being catalytically active.

Aptamers are specific ligand-binding nucleic acid receptors (ssDNA, RNA, modDNA or modRNA) which can be isolated by screening the 'shape-space' of vast combinatorial libraries of single stranded nucleic acids by in vitro selection. In vitro selection of aptamers can be achieved by contacting a nucleic acid library with a target, e.g. a protein, and partitioning the bound nucleic acids from the remainder of the nucleic acid mixture. Partitioning can be achieved by retaining the nucleic acid/protein complexes on nitrocellulose filters (Tuerk and Gold, Science 249 (1990), 505-510), immobilizing the target prior to incubation with the library on a solid support and removing non bound species through washes with an appropiate buffer (Nieuwielandt et. al, Biochemistry 34 (1995), 5651-5659) or any other method which allows the separation of the target/nucleic acid complexes from unbound nucleic acid species. After isolation of the bound species DNA can be amplified using PCR or in the case of RNA reverse transcription, PCR and transcription. This cycle can be repeated several times to yield a library with increased affinity for the target. Subsequently individual aptamer sequences can be identified by cloning and sequencing the PCR products using standard methods.

Nucleic acid aptamers for far more than a hundred different targets have been described showing that aptamers can now routinely be obtained for almost every desired target (for reviews see e.g.: Osborne and Ellington, Chem. Rev. 97 (1997), 349-370; Famulok and Jenne, Curr. Opin. Chem. Biol. 2 (1998), 320-327).

The term ribozymes is used in the present invention for catalytically active nucleic acids that act on an intracellular target thereby modifing its functions. Not included in the present invention are naturally occuring ribozymes, such as hammerhead ribozymes, hairpin ribozymes or derivatives therof which act by Watson Crick binding to nucleic acid substrates and phosphodiester cleavage and are used for example in gene therapeutic approaches (Bramlage, Tibtech 16 (1998), 435-438). Ribozymes with new catalytic properties can be isolated using in vitro selection experiments. For example ribozymes have been isolated which are able to catalize RNA alkylation, amide bond formation or porphyrin metalation (Breaker, Chem. Rev. 97 (1997), 371-390).

Aptazymes can be viewed as chimeras between a catalytic RNA and an aptamer. These chimeras can be rationally designed or selected in vitro from certain combinatorial libraries (see:Robertson and Ellington, Nature Biotechnol. 17 (1999); 62-66). Usually, the aptameric domain of an aptazyme is used as a regulatory module for allosteric ribozyme activation or inhibition (Tang and Breaker, Chem. Biol. 4 (1997), 453-459; Tang and Breaker, RNA 3 (1997), 914-925; Tang and Breaker, Nucleic Acids Res. 26 (1998), 4214-4221; Araki et al., Nucleic Acids Res. 26 (1998), 3379-3384). Likewise, and consequently, if an aptazyme is selected to be affected by a regulatory molecule it contains a binding domain for this molecule and thus can also act like an aptamer.

Intramers are functional nucleic acids (aptamers, aptazymes etc.) that are delivered exogenously or endogenously into procaryotic or eucaryotic cells and bind, modulate or enzymatically modify an intracellular target.

Modulation of an intracellular target can be, for example, the inhibition or stabilization of a protein/protein interaction or protein-domain/protein interaction, or protein/nucleic acid interaction, or protein/small molecule interactions etc. Modulation can also include activation or inhibition of enzymes via different mechanisms such as sequestering the substrate(s), binding to the active site, induction of conformational changes, allosteric regulation etc. Modulation of an intracellular target can occur both by non-covalent interactions or covalent modifications such as biotinylation (Wecker et al., RNA 2 (1996), 982-94). Further methods developed for example for antibody based approaches could be adapted to the functional nucleic acid system in which case the aptamer could be conjugated to a molecule that is able to chemically inactivate the bound target. For example, the intracellular functional nucleic acids are tagged via a short annealing sequence which is covalently attached to the dye malachit green (MG). These short oligonucleotides could be applied exogenously. MG absorbs light at a visible wavelength which is not significantly absorbed by cells. After irradiation with a laser the MG becomes activated and generates short lived hydroxyl radicals which inactivate proteins most likely by modifying amino acid side chains ["Chromophore assisted laser inactivation (CALI)" see: Liao et al., Proc. Natl. Acad. Sci. 91 (1994), 2659-63)]. The inactivation process occurs only at a very short distance from the dye moiety. Thus, the targeted protein or subunit can generally be damaged without affecting its neighbours. This has two advantages: First, the aptamer would not neccessarily have to bind to the active site of the protein but could interact with a neighbouring epitope that might be better suited for binding to a nucleic acid. Even though the bound epitope is distant from the active site the protein will still be specifically inactivated. Second, as the protein is irreversibly inactivated and the aptamer can be constitutively expressed, several pulses of irradiation could inactivate high abundance proteins that are initially present at higher copy numbers than the specific aptamer.

Intracellular targets include small molecules, biological cofactors, natural polymers such as proteins, protein domains, peptides, and nucleic acids, glycoproteins, hormones, receptors, protein complexes, protein/nucleic acid complexes, toxins, viruses, and other compounds that are naturally found inside cells.

Phenotye can be for example the induction or repression of the transcription of a certain gene, processes linked to signal transduction cascades such as adhesion in response to certain stimuli, differentiation of cells, apoptosis etc.

Phenotypic selection is a process in which nucleic acid libraries are introduced into cells by various methods known to a person skilled in the art (electroporation, lipofection, expression with viral vector systems, expression from plasmids containing RNA polymerase promotors etc.). Those cells containing a functional nucleic acid acting on a target which is involved in a certain phenotype are separated on the basis of their altered phenotype from the bulk cellular material which do not show an altered phenotype. Cells used in the phenotypic selection include procaryotes, yeast, insect- or plant-cells and preferentially mammalian cells (e.g. CHO, HeLa, COS, MDCK, 293, WI38 and Jurkat E6 cells). If a clear separation on the basis of the altered phenotype is not possible or difficult, reporter constructs can be applied which allow a more definite or better discrimination. In a positiv phenotypic selection experiment, for example the induction of a cellular promotor or enhancer/promotor construct, a reporter gene which allows the identification of the altered phenotype (here the induction of the promotor) is inserted under the control of the inducible promotor in the reporter construct. Reporter genes used in this type of selection are for example resistance genes like aminoglycoside phosphotransferase (Southern and Berg, J. Mol. Appl. Genet. 1 (1982), 327-341) which inactivates aminoglycoside antibiotics (neomycin, G418) making cells resistant to this drug. Consequently, if the inducible promotor is turned on through the action of a functional nucleic acid (fNS), the cells harbouring the gene for the functional nucleic acid will survive the presence of the drug and the genes for the fNS can be identified. For example mammalian promotors which are dependent on activation via the transcription factor NF□B could be activated by inhibiting the I□B proteins which normally prevent the import of NF□B into the nucleus (Rolfe et al., J.Mol.Med. 75 (1997),5-17; Fuchs et al., Oncogene 17 (1998), 1483-90). Other examples of promotors which are regulated by an inhibitory mechanism are bacterial transcripton units, for example the E.coli lac operon regulated by the lac repressor (Gilbert and Muller-Hill, Proc.Nat.Acad.Sci. 56 (1966),1891-1898).

Negative phenotypic selection can occur for example by inhibiting the induction of a promotor. In this case the reporter gene could be a toxin (e.g. Herpes simplex virus thymidine kinase (Fife et al., Gene Ther. 5 (1998), 614-620) or diphteria toxin (Massuda et al., Proc.Natl.Acad.Sci. U.S.A. 94 (1997), 14701-14706)), which promotes the death of cells where no functional nucleic acid inhibits the cellular signal transduction pathway which induces the promotor. Inducible genes include bacterial, viral and eukaryotic transcriptional units, e.g. in the arabinose operon (Wilcox et al., J.Biol.Chem 249 (1974), 2946-2952), the vaccinia virus early and late genes (Moss, Annu. Rev. Biochem. 59 (1990), 661-688) or promotors of immunglobulin genes (Staudt et al., Ann. Rev. Immunol. 10 (1991), 373-398). The promotors used in the phenotypic selections may be naturally occuring promotor/enhancer elements or any combinations or modifications thereof.

Reporter constructs can include positive or negative phenotypic selections. For example, negative phenotypic selection can occur via the expression of a toxin (such as the diphteria toxin) under the control of an inducible cellular promotor such as the interleukin-2 promotor in T-lymphocytes. These gene constructs can be introduced into cells e.g. via plasmids and the expression of the toxin-gene will be driven by the cell's natural promotor-inducing machinery. Only in those cells in which a functional nucleic acid inhibits the induction of the toxin-promotor the toxin will not be expressed and the cells survive and can be selected.

The vectors (vector elements) and protocols for cloning, transfecting, transient gene expression and obtaining stable transfected cell lines are well known and described in the literature (Maniatis et al. (1989) Molecular Cloning: A Laboratory Manual, Cold Spring Harbor Laboratory Press, Cold Spring Harbour, New York; Spector et al., Cells: A Laboratory Manual (1998), Cold Spring Harbor Laboratory Press, Cold Spring Harbor, New York). Vectors for the expression of the nucleic acid libraries can be any commonly used constructs to introduce foreign genes into cells, for example linear PCR fragments, plasmids, DNA viruses like M13 or vaccinia, RNA viruses like retroviruses or the Semliki Forest virus. The genes for the nucleic acid library are placed under the control of suitable promotors, preferentially those, which allow high level transcription of the nucleic acid genes.

Examples are but are not limited to polymerase III dependent promotors such as yeast RNase P promotor (Good and Engelke, Gene 151 (1994),209-214), mammalian U6 or tRNA^{met} promotors (Bertrand et al., RNA 3 (1997),75-88), retroviral long terminal repeats (Rossi, Tibtech 13 (1995), 301-306), T7 RNA polymerase promotors as described in Example 1 or the subgenomic promotor for the Semliki Forest virus replicase (Tubulekas et al., Gene 190 (1997), 191-195).

Vectors used as reporter constructs can be plasmids (e.g. pUC18, pYES2, pCDM8) or viral genomes such as adenovirus, AAV or retroviral vectors (Moloney murine leukemia virus (MoMuLV), gibbon ape leukemia virus (GaLV), etc. Using stably transfected reporter cell lines is optional but provides a reliable system to select for cells which survive the negativ selection in the presence of a toxin.

Target validation is the identification of a given cellular target as a key molecule responsible for a given phenotype. It involves the intracellular application of a nucleic acid modulator which affects the target to investigate its role in the complex network of cellular components. The preferential goal is to investigate whether or not the target is responsible for a certain cellular status or disease state and thus is a good drug target.

Target identification is the linkage of a cellular component to a given phenotype regardless whether its biological function is known or not. Target identification can occur by means of a functional nucleic acid modulator which has been identified by phenotypic selection; the target affected by such a modulator is likely to represent the or a key molecule responsible for a certain phenotype. The target can be identified through its interaction with the functional nucleic acid. For example the yeast three-hybrid system, a modification of the two-hybrid system, is used to detect RNA-protein interactions. The three-hybrid system utilizes an RNA hybrid as an intermediate between two hybrid proteins. The interaction of the three components serves to reconstitute transcriptional activation of reporter genes in a mechanism similar to the two-hybrid system (SenGupta et al., Proc. Natl. Acad. Sci. 93 (1996), 8496-8501). The functional nucleic acid cloned in the RNA hybrid will interact with its target protein expressed as part of a protein-hybrid library of cellular proteins. Therefor the reportergenes will be expressed in those yeast cells which harbour both, the functional nucleic acid and its target, and the plasmids with the target genes can be isolated.

Another approach is to identify the target via complementation cloning as outlined in Example 2. This technique has been applied successfully to isolate proteins which functionally complement cell lines bearing mutatated endogenous genes, for example complementation cloning of NEMO a component of the IkB kinase complex (Yamaoko et al., Cell 93 (1998), 1231-1240).

Highly specific nucleic acid modulators of cellular target molecules can be routinely obtained for example by application of the technologies SELEX, aptazymes, two- and three-hybrid technologies, the phenotypic selection method described herein etc. The resulting nucleic acid modulators can be brought into cells exo- or endogenously by methods such as electroporation, lipofection, expression with viral vector systems, expression from plasmids containing RNA polymerase promotors etc. Functional nucleic acids can act on their target molecule by different mechanisms; they can either bind their target molecule in an antibody-like fashion thereby modulating its biological function. For example, chemically modified nuclease resistant aptamers have been selected for binding to extracellular growth factors such as basic fibroblast growth factor (bFGF; see: [Jellinek et al., Proc. Natl. Acad. Sci. USA 90 (1993), 11227-11231; Jellinek et al., Biochemistry 34 (1995), 11363-11372.]), human keratinocyte growth factor (hKGF; see: [Pagratis et al., Nature Biotech. 15 (1997), 68-73]), platelet derived growth factor (PDGF; see: [Green et al., Biochemistry 35 (1996), 14413-14424]), or vascular endothelial growth factor (vEGF; see: [Green et al., Chem. Biol. 2 (1995), 683-695; Jellinek et al. Biochemistry 33 (1994), 10450-10456; Ruckman et al., J. Biol. Chem. 273 (1998), 20556-20567]). These aptamers blocked the binding of their extracellular target to its natural protein receptor thus modulating the growth factor's biological function.

While it is state of the art to modulate the biological function of an extracellular protein, a central aspect of the present invention is the exploitation of the huge potential of high affinity and highly specific nucleic acid reagents to modulate a given target for intracellular applications. That this can indeed be done was shown representatively in the system exemplified in Example 1:

An approach is described which is based on integrin cytoplasmic domain recognition by synthetic ligand-binding RNA-aptamers isolated from a combinatorial RNA library. A vaccinia virus-based RNA expression system enabled high-level cytoplasmic expression of RNA aptamers (intramers) directed against the intracellular domain of the β2 integrin LFA-1, a transmembrane protein which mediates cell adhesion to intercellular adhesion molecule-1 (ICAM-1). In two different cell types, cytoplasmic expression of integrin-binding intramers reduced inducible cell adhesion to ICAM-1. The aptamers specifically target, and thereby define a functional cytoplasmic subdomain important for the regulation of cell adhesion in leukocytes.

Integrins are versatile heterodimeric transmembrane proteins which mediate adhesive interactions with extracellular matrix and cell specific counter-receptors. They are implicated in diverse biological processes including apoptosis, cell-cycle regulation, cell migration, bloodclotting, memory and leukocyte function (Hynes Cell 69 (1992), 11-25). The cytoplasmic domains of integrins play important roles in regulating cell adhesion (Diamond and Springer Curr. Biol. 4 (1994), 506-517; Dedhar and Hannigan Curr. Opin. Cell Biol. 8 (1996), 657-669), probably by linking signaling events inside the cell to the extracellular domains, but the precise mechanisms are unclear. The β2 integrin LFA-1 (αLβ2, CD11a/CD18) mediates adhesion of leukocytes in immune and inflammatory responses by binding to cellular ligands (Lub et al., Immunol. Today 16 (1995), 479-483), the intercellular adhesion molecules ICAM-1, -2, or -3. A large body of data implicates the cytoplasmic domain of the β2-chain in the regulation of LFA-1 mediated adhesiveness, presumably through its interactions with intracellular proteins and/or the membrane-proximal cytoskeleton (Stewart et al. J. Cell Biol. 140 (1998), 699-707), and some candidate cytoplasmic ligands have recently been identified (Hibbs et al., J. Exp. Med. 174 (1991), 1227-1238; Pavalko and LaRoche, J. Immunol. 151 (1993), 3795-3807; Sharma et al., J. Immunol. 154 (1995), 3461-3470; Kolanus et al., Cell 86 (1996), 233-242). To gain further insights into these mechanisms a system was devised allowing the specific inhibition of the LFA-1 mediated inside-out signal transduction in vivo. The objectives were the selection of aptamers which bind to the cytoplasmic domain of CD18, the development and application of a system allowing high-level expression of aptamers within the cytoplasm of leukocytes, and investigations into their biological effects in the context of the living cell.

Briefly, as detailled in Example 1 below, the inventors were able to
a) isolate RNA aptamers which bind to an immobilized peptide corresponding to the cytoplasmic domain of the integral transmembrane protein LFA-1 (CD18, β2-integrin)
b) show that the aptamers recognize their target specifically even when cloned into a different sequence context in an RNA expression cassette. This cassette was derived from a T7 RNA polymerase dependent RNA expression system (Fuerst and Moss, J. Mol. Biol. 206 (1989), 333-348) and contained a 5'-stem-loop structure which has previously been shown to stabilize RNA molecules in the cytoplasm by preventing their degradation. Furthermore, the 3'-end contained the Tφ-terminator hairpin loop motif as a terminator for T7 RNA polymerase dependent transcription. Another example for a cytoplasmic system, which might be suitable for RNA expression, is based on the RNA dependent RNA Replicase of viral vectors like Semliki Forest virus (SFV) (Tubulekas et al., Gene 190 (1997), 191-195). Most importantly, it was shown that the aptamer transcripts resulting from the T7 RNA polymerase-expression cassette template were correctly terminated and fully retained their binding activity to CD18cyt. Incorrectly terminated aptamer sequences which contained additional sequence information from the viral genome for binding to CD18cyt were also tested. It was found that this undesired additional sequence information resulted in a severely reduced binding affinity of these aptamer variants to CD18cyt in vitro probably due to interfering with correct aptamer folding. This result establishes two very important and surprising points: i) it shows that it is possible to express the aptamer in the context of additional sequence information such as stabilizing sequence motifs or RNA localization signals etc. which is crucial for broad application of endogenous aptamer-expression ii) it shows that it is possible to engineer small, compact aptamers selected in vitro in a modular fashion providing the aptamers with additional properties ensuring proper and efficient intracellular applicability without affecting aptamer-binding to the target.
c) show that the selected aptamers specifically recognize endogenous LFA-1 in crude Jurkat E6 cell lysate. They do so also in the context of the RNA expression cassette.
d) develop a vaccinia virus-based RNA expression system which enabled high-level cytoplasmic expression of RNA aptamers directed against the cytoplasmic domain of the integrin LFA-1.
e) alter the phenotype of two different cell types, Jurkat E6 and peripheral blood mononuclear cells (PBMC) by cytoplasmic expression of aptamers binding the cytoplasmic domain of LFA-1 in these cells; in this case cytoplasmic aptamer expression specifically reduced inducible cell adhesion to intercellular adhesion molecule 1 (ICAM-1). This result is unexpected and represents one of the central aspects of this invention for several reasons: i) for the first time the intracellular target is a protein or protein domain without any known natural affinity to nucleic acids. The novel and unexpected aspect of this finding is that the in vitro selected aptamer specifically finds the target it has been selected for in an intracellular compartment which is very different from other compartments because it contains a huge number of professional RNA-binding proteins (such as helicases, ribosomal proteins, hnRNP proteins etc.) which exist in high copy numbers of several million per cell. These professional RNA-binding proteins can bind with very high affinity (K_{d} 10⁻⁹-10⁻⁸ M) to special RNA motifs but can also interact with RNAs in a more sequence independent manner with still relatively high affinities [e.g. the hnRNP proteins A1 and C1; K_{d} 10⁻⁷-10⁻⁶M; see: Nagai and Mattaj (Eds.), RNA-Protein Interactions (1994), Oxford University Press, 138-140]. Despite the expectation that these professional RNA-binding proteins compete with the cognate aptamer-target for binding to the aptamer it was shown here that the aptamer can find its target inside the cell and change the cell's phenotype. ii) although the in vitro affinity of the aptamer to its cognate CD18cyt protein domain was relatively low (K_{d} 10⁻⁶ - 5 x 10⁻⁷ M) it recognizes its target with unexpected specificity. This shows that this system is not restricted to targets which are recognized by an aptamer with affinities that are significantly higher than the unspecific interactions between a professional RNA-binding protein and a nucleic acid. This opens up the exciting possibility to generally apply aptamers even if they bind their targets with relatively low affinity. iii) it has been shown for the first time that functional nucleic acid ligands can act in cellular sub-compartments in which nucleic acids normally do not appear. In this particular case the aptamer finds its target even though it is anchored to the negatively charged plasma membrane including its associated compact cytosceletal protein cortex. It could not be expected in advance that the target would be accessible by the aptamer at these sites.
f) map the binding site of the aptamers to the cytoplasmic domain of LFA-1 (CD18cyt) by employing synthetic peptides comprising overlapping regions of the cytoplasmic domain of LFA-1. Thereby, the aptamer binding site on CD18cyt was defined as functionally important for stimulated cell adhesion of LFA-1 to ICAM-1.

Most of our current knowledge regarding factors and mechanisms that control integrin activation so far stems from mutational analyses of integrin domains. This study shows that it is now feasible to address this issue by retaining the genetic integrity of the wild-type CD18 cytoplasmic domain. For the first time it was shown that the intracellular expression of CD18cyt-specific aptamers can affect the functional state of endogenous LFA-1 molecules. Futhermore, evidence is provided that these "intramers" target a specific subdomain of the β2 cytoplasmic tail implicating the importance of this region for the proper function of the endogenous LFA-1 receptor with respect to its adhesion to ICAM-1.

Current state of the art technologies are able to produce nucleic acid ligands which bind almost any given target with high affinity and specificity. Extension to automation of the selection procedures may even speed up the generation of such compounds. Although the process of in vitro selection of nucleic acid aptamers is currently done largely manually, the first fully automated selection protocoll has been reported and it is suggested that in the future it would potentially allow the isolation of hundreds of nucleic acid ligands within a few days making it much more efficient than screening small molecule compound libraries for possible effectors (see Cox et al., Biotechol. Prog. 14 (1998), 845-50).

The method described above employs these specific nucleic acid modulators to determine the role of an intracellular component in the complex intracellular regulatory network or to find out whether it is the causal reason for a given phenotype. Furthermore, it is a powerful system which enables the validation of potential drug targets.

The intracellular application of functional nucleic acid ligands can easily be achieved. Depending on the suitability and the questions asked, the functional nucleic acids can be delivered either endogenously or exogenously. In the latter case, chemically modified forms that prevent the degradation of the functional nucleic acid are preferentially applied. Stabilizing modifications include, for example, RNA molecules in which the 2'-hydroxy function of pyrimidine bases is substituted by a 2'-amino or 2'-fluoro residues. Endogenous transcription expression systems such as the one described in Example 1 allow the efficient intracellular expression of functional nucleic acids. Depending on the application and site of action, appropriate vectors and promotors known to the expert are choosen. The functional nucleic acids can be engineered with additional nucleic acid domains to ensure proper function and localization.

In a second aspect the present invention relates to methods for the expression of randomized nucleic acid libraries inside cells for the identification of functional intramers which alter the phenotype of the cell in which they are expressed. That this is feasible is a direct consequence of the effectiveness of the system as described above. This target identification approach is useful for directly establishing a cellular component as important for or involved in a particular phenotype such as a certain status associated with a disease. Based on current knowledge it can be estimated that a nucleic acid library expressed inside cells contains individual functional nucleic acids which are able to specifically recognize a cellular component. Based on the findings described in the first part of this invention it can also be expected that this specific recognition is maintained or is generally possible within the cellular compartment. For example, the library should contain molecules which are able to bind or modify activators or inhibitors of signal transduction pathways thereby modulating phenotypes associated with the cascade. In such a case the cell containing the functional nucleic acid can be isolated by phenotypic selection (see definition above). If a clear separation on the basis of the altered phenotype is not possible or difficult, reporter constructs can be applied which allow a more definite or better discrimination. This can be done without prior knowledge regarding the nature of the target or its biological function. Thus, the first step of this process is the phenotypic selection. The nucleic acid library is introduced into the cell for example via expression vectors. Phenotypic selection can be achieved preferentially via reporter constructs either by positive or negative selection. Positive phenotypic selection could occur, for example, by expression of an appropriate cell surface marker and subsequent affinity isolation of the cell with a surface marker-specific antibody. Negative phenotypic selection can occur via the expression of a toxin (such as the diphteria toxin or herpes simplex virus thymidine kinase) under the control of an inducible cellular promotor. These gene constructs can be introduced into cells e.g. via plasmids and the expression of the toxin-gene will be driven by the cell's natural promotor-inducing machinery. Only in those cells in which a functional nucleic acid inhibits the induction of the toxin-promotor the toxin will not be expressed and the cells survive and can be selected. After enrichment of the selected phenotype the functional nucleic acid modulators are identified by sequencing and tested individually for functionality. Once the functional nucleic acid has been identified and established it can in turn be applied to identify its intracellular interactor. This can be achieved, for example, by standard methods for investigating protein/nucleic acid interactions such as screening proteins expressed from cDNA-libraries according to the yeast three hybrid system to detect RNA/protein interactions in vivo (see: SenGupta et al., Proc. Natl. Acad. Sci. 93 (1996), 8496-8501). Preferentially, the identification occurs via complementation in homologous cells. By expression of cDNA libraries in homologous cell systems clones can be isolated which complement the signal transduction cascade modulated by the functional nucleic acid. At the same time, reporter systems can be constructed in cells in which the aptamer/target interaction is confirmed by systems such as the three hybrid system (see: SenGupta et al., Proc. Natl. Acad. Sci. 93 (1996), 8496-8501) or the approach described in Example 2 (see below) for the positive selection incorporating negative selection proofreading. This provides a dual read-out for the reliable identification of those proteins which play a key role for a particular phenotype in a wild-type context.

In addition, the phenotypic selection system also identifies nucleic acid inhibitors which could simply be applied for target validation (see first part of this invention). This method, thus, significantly contributes to the field of functional genomics/proteomics because it identifies targets directly in an unaltered cellular status by their function rather than their genes or their presence in a cell. Therefore this method of the present invention is superior over the current state of the art.

The identified functional nucleic acids can serve as drug leads or can be used as therapeutics themselves, particularly in gene therapeutic approaches. Or in a pharmaceutical composition comprising a functional nucleic acid identified by the methods of the present invention which is capable of binding to and modifying the function of the functional intracellular target, optionally in combination with a suitable pharmaceutical carrier.

Examples of suitable pharmaceutical carriers are well known in the art and include phosphate buffered saline solutions, water, emulsions, such as oil/water emulsions, various types of wetting agents, sterile solutions etc. Such carriers can be formulated by conventional methods and can be administered to the subject at a suitable dose. Administration of the suitable compositions may be effected by different ways, e.g. by intravenous, intraperitoneal, subcutaneous, intramuscular, topical or intradermal administration. The route of administration, of course, depends on the nature of the disease and the kind of compound contained in the pharmaceutical composition. The dosage regimen will be determined by the attending physician and other clinical factors. As is well known in the medical arts, dosages for any one patient depends on many factors, including the patient's size, body surface area, age, sex, the particular compound to be administered, time and route of administration, the kind of the disease, general health and other drugs being administered concurrently.

Preferably, the functional nucleic acid (e.g. intramer) is administered directly to the target site, e.g., by biolistic delivery, as a colloidal dispersion system or by catheter to a site in artery. The colloidal dispersion systems which can be used for delivery of the pharmaceutical composition include macromolecule complexes, nanocapsules, microspheres, beads and lipid-based systems including oil-in-water emulsions, (mixed) micelles, liposomes and lipoplexes. The preferred colloidal system is a liposome.

The composition of the liposome is usually a combination of phospholipids and steroids, especially cholesterol. The skilled person is in a position to select such liposomes which are suitable for the delivery of the desired nucleic acid molecule. Organ-specific or cell-specific liposomes can be used in order to achieve delivery only to the desired site. The targeting of liposomes can be carried out by the person skilled in the art by applying commonly known methods. This targeting includes passive targeting (utilizing the natural tendency of the liposomes to distribute to cells of the RES in organs which contain sinusoidal capillaries) or active targeting (for example by coupling the liposome to a specific ligand, e.g., an antibody, a receptor, sugar, glycolipid, protein etc., by well known methods). In the present invention monoclonal antibodies are preferably used to target liposomes to specific sites via specific cell-surface ligands.

In a still more preferred embodiment the functional nucleic acid molecule (e.g. intramer) is integrated into a recombinant vector, preferably an expression vector. In order to achieve expression only in the target organ, the functional nucleic acid molecule encoding DNA is operably linked to a tissue specific promotor and used for gene therapy. For example, it can be ligated to the CD4 gene promotor (Zhao-Emonet et al., Biochim. Biophys. Acta. 1442 (1998), 109-119), secretory leukoprotease inhibitor gene promotor (Robertson et al., Cancer Gene Ther. 5 (1998), 331-336) or carcinoembryonic antigen promotor (Fichera et al., Dis. Colon. Rectum. 41 (1998), 747-754). Other tissue specific promotors are well known to those skilled in the art.

Preferred recombinant vectors are viral vectors, e.g. adenovirus, herpes virus, vaccinia, or, more preferably, an RNA virus such as a retrovirus. Even more preferably, the retroviral vector is a derivative of a murine or avian retrovirus. Examples of such retroviral vectors which can be used in the present invention are: Moloney murine leukemia virus (MoMuLV), Harvey murine sarcome virus (HaMuSV), murine mammary tumor virus (MuMTV) and Rous sarcoma virus (RSV). Most preferably, a non-human primate retroviral vector is employed, such as the gibbon ape leukemia virus (GaLV), providing a broader host range compared to murine vectors. Since recombinant retroviruses are defective, assistance is required in order to produce infectious particles. Such assistance can be provided, e.g. by using helper cell lines that contain plasmids encoding all of the structural genes of the retrovirus under the control of regulatory sequences within the LTR. Suitable helper cell lines are well known to those skilled in the art. Said vectors can additionally contain a gene encoding a selectable marker so that the transduced cells can be identified. Moreover, the retroviral vectors can be modified in such a way that they become target specific. This can be achieved, e.g., by inserting a polynucleotide encoding a sugar, a glycolipid, or a protein, preferably an antibody. Those skilled in the art know additional methods for generating target specific vectors. Further suitable vectors and methods for in vitro- or in vivo-gene therapy are described in the literature and are known to the persons skilled in the art; see, e.g., WO 94/29469 or WO 97/00957.

A pharmaceutical composition can contain a compound which has the same or an even increased effect compared to the original functional nucleic acid (intramer) identified by the methods of the present invention. A strategy for increasing the stability and improving the binding specifities of the functional nucleic acids identified by the methods herein has been described as post-SELEX modification and deconvultion of post-SELEX modified aptamers (Eaton, Cur. Opin. Chem. Biol. 1 (1997), 10-16). After identification of the functional nucleic acid a secondary nucleic acid library is derived from the parental sequence by combinatorial chemical synthesis and random incorporation of modified nucleosides. For example, the purine residues could be synthezised with mixtures of 2'-OH and 2'-O-methyl substituted nucleoside phosphoramidites. The ratios can be varied and will depend on the desired application. Modified nucleosides which can be included in the chemical synthesis are well known to a person skilled in the art and include, for example, modifications at the 5-position of pyrimidines, 8-position of purines or the 2'-position of all nucleotides. Modifications include but are not limited to vinyl-, prenyl-, fucose-, biotin- or arginine-groups, adding additional chemical properties to the nucleic acid. In a second SELEX-experiment the modified secondary library is contacted with the ligand and those clones identified which exhibit the same or increased binding properties compared to the parental sequence. The identification of the modified positions in the winning oligonucleotides can be achieved, for example, via alkaline hydrolysis interference experiments because 2'-O-methyl substitutions protect the adjacent phosphodiesterbond from alkaline hydrolysis. Using this method secondary aptamers against vascular endothelial growth factor (VEGF) have been identified which bind VEGF with a 17-fold increased affinity probably due to additional hydrophobic contacts mediated via the methyl substitutions and 8 fold increased stability against degradation (t_{1/2} = 171h) in undiluted rat urin (Green et. al., Cur. Biol. 2 (1995), 683-695). Thus increased efficiency (e.g. better affinities or better cellular uptake) can be added to the nucleic acids by chemical substitutions and the stabilized forms could be useful for the exogenous delivery of the functional nucleic acids as pharmaceutical formulations, encompassing the need to introduce the genes for the functional nucleic acids into the target cells.

Moreover the functional nucleic acids could be used as functional leads to screen small molecule libraries for new drugs. If the functional nucleic acid acts for example as a inhibitor of an enzyme through binding the active site, this interaction can be used to screen for small molecules which bind at the same site and act as a mimetic of the functional nucleic acid. Further binding to the same site suggests that the molecule can act in the same fashion as the functional nucleic acid, namely inhibiting the enzymes function. Those small molecule mimetics could be identified, for example, by variations of the three hybrid system and reverse two hybrid system. As described in the target identification process two hybrid proteins and one hybrid RNA form a complex in the three hybrid approach and activate gene transcription in yeast. In the reverse two hybrid system reported by Leanna and Hannik, Nucleic Acid Res. 24 (1996), 3341-3347, the reportergen CHY2 is transcribed through the activation of transcription mediated by the interaction of two hybrid proteins. This results in cell death of yeast cells in the presence of cycloheximide. In a three hybrid system with similar reporter constructs designed for the interaction of the functional nucleic acid and in this example the target enzyme only the inhibition of the assembly of the three hybrid components will promote cell survival. Consequently this assay can be used to screen small molecule libraries for mimetics which compete with the functional nucleic acid for binding to the target enzyme, therefor preventing the formation of the transcription activating complex. This compounds can further be assayed for their ability to functionally inhibit the enzyme and can serve as drugs or drug leads.

The functiona nucleic acids of the invention or the compounds described above are particularly useful for the preparation of a medicament for gene therapy. They could be used besides other examples for the treatment of diseases which have been proposed to be targets for the application of intracellular antibodies, for example HIV infections (Chen et al., Human Gene Ther. 5 (1994), 595-601) or leukemias, breast and ovarian carcinomas (Richardson and Marasco, Tibtech 13 (1995), 306-310)

Finally, the functional nuclei acids which are identified by the method according to the present invention can be continued in a kit for the diagnosis of diseases which are associated with a particular intracellular target as discussed above. Such a kit can be used in order to determine whether this target is present, has an abnormal concentration, etc.

### Example 1: Cytoplasmic RNA-modulators of an inside-out signal transduction cascade

### (a) In vitro selection of CD18-binding RNA aptamers.

An RNA library with a complexity of 5·10¹⁴ different molecules was synthesised by *in vitro* T7-transcription from the PCR-amplified synthetic DNA pool MF76.1: 5'-*TC TAATACGACTCACTATAGGGCGCTA* AGTCCTCGCTCA-N40-ACGCGCGACTCGGATCCT-3'; primer *MF39.1:* 5'-TC TAATA *CGACTCACTATAGGGCGCTAA* GTCCTCGCTCA-3' (italic: T7-promotor, bold: BamHI restriction site); primer Mic20.1: 5'-GTA**GGATCC**GAGTCGCGCGT -3' (bold: BamHI restriction site) as described previously (Klug et al., Proc. Natl. Acad. Sci. USA 94 (1997), 6676-6681). CNBr-activated sepharose was derivatized with synthetic peptides (CD18cyt, MF2G), or blocked with Tris-HCl (pH 8.0) alone according to the manufacturers protocol (Pharmacia Biotech AB, Uppsala, Sweden). For the pre-selection, 50-75 µl of either Tris-blocked- or MF2G-derivatized Sepharose was incubated with 2-3 nmol α-³²P-labeled RNA in 150 µl of binding buffer (buffer B: 4.3 mM K₂HPO₄, 1.4 mM NaH₂PO₄, 150 mM NaCl₂, 1.0 mM MgCl₂, 0.1 µM CaCl₂) for 1 h at 23° C. The slurry was transferred into a plastic column and eluted in small fractions (75 µl) with buffer B. For the actual selection the initial four fractions were precipitated and incubated with 25-50 µl CD18cyt-Sepharose (4.5 mg/ml gel) in 100 µl buffer B for 1 h at 23° C. Removal of non-binding RNAs was achieved by washing with 200 Sepharose-volumes of binding buffer (5-10 ml). Bound RNAs were eluted by washing with buffer B adjusted to 6 M guanidinium-HCl. Eluted fractions were quantified by Cherenkov counting, ethanol-precipitated in the presence of 10-20 µg glycogen, and reverse transcribed as described previously (Famulok, J. Am. Chem. Soc. 116 (1994), 1698-1706); cDNA was PCR-amplified, followed by in vitro transcription. For the first cycle 29 nmol RNA and 120 µl CD18cyt-Sepharose in a total volume of 475 µl were used. The RNA library was subjected to 11 cycles of *in vitro* selection by binding to a 46-mer peptide corresponding to the complete cytoplasmic domain of CD18 (CD18cyt) immobilised on a sepharose matrix (Figure 1A). After cycle 11, binding sequences were PCR-amplified with primers Mic20.1 and SK40.4 (5'-TC*TAATACGACTCACTATAGGG***CTGCAG**AGTCCTCGCTCA-3'; italic: T7-promotor, bold: PstI restriction site) to introduce a Pst I restriction site, cloned, and sequenced as described previously (Famulok, J. Am. Chem. Soc. 116 (1994), 1698-1706).From the enriched pool, 80 aptamers were cloned and sequenced. Based on sequence motifs these aptamers can be subdivided into either four classes or orphan sequences without any obvious relationship to each other.

### (b) Construction of the cytoplasmatic aptamer-expression-cassette

Four sequences were chosen for the cytoplasmic expression studies. Three of them, D20, D28, and D31 are CD18-binders, the fourth sequence, D42, is a non-binding negative control sequence of the same length and with primer binding sites identical to the specific aptamers (Figure 1B). First, a T7-RNA expression cassette (TR) vector was constructed where the T7 promoter is located upstream of aptamer-coding DNA inserted between 5'- and 3'-stem-loop structures which serve as RNA-stabilising motifs and are required for correct termination of the T7-transcripts (Fuerst and Moss, J. Mol. Biol. 206 (1989), 333-348) (Figure 2A). The sequence of the T7-RNA expression cassette, TR, shown here without inserted aptamer (5'-**GTTAAC**-**GCATGC***TAATACGACTCACTATAG* GGAGACCACAACGGT TTCCC**GGG**CGCAAGTT**ACTAGT-TGGCCA-AGATCT-TAATTAA**TAGCATAACCCCTTGG GGCCTCTAAACGGGTCTTGAGGGGTTTTTTGCT**GTCGAC-GCGGCCGC**-3'; bold: restriction sites in the order in which they appear HpaI, SphI, XmaI, Spel, Ball, BglII, PacI, SalI, NotI; italic: T7-promotor; underlined: stabilizing 5'stem-loop and 3' Terminator Tφ for correct termination of the TR-aptamers) was inserted into the vector pTkg (Romeo and Seed, Cell 64 (1991), 1037-1046; Falkner and Moss, Virol. 62 (1988), 1849-1854) via Hpa1 and Not1 restriction sites resulting in the transfer TR vector. Aptamer-encoding sequences were inserted via the XmaI and PacI restriction sites (TR-aptamer; for an overview see Figure 2A). The resulting transcripts are called TR-aptamers. As negative controls, a TR vector lacking the aptamer sequence was also used and one that expressed the non-binding negative control sequence TR-D42.

### (c) In vitro binding of aptamers and TR-aptamers

*In vitro* binding to the CD18cyt-peptide immobilized on a sepharose matrix was confirmed for several aptamers (Figure 1B) using zonal elution quantitative affinity chromatography assays as described in Dean et al., Affinity chromatography a practical approach (1991), pp 169-174, IRL Press, Oxford, UK. The binding affinity of monoclonal aptamers to the immobilised CD18cyt-peptide was between 500 and 6000 nM (Table 1). No binding of individual aptamers to un-derivatised sepharose was observed. Importantly, correctly terminated TR-aptamers were confirmed to exhibit the same *in vitro* binding behaviour to the CD18cyt-peptide when expressed in the context of the flanking stem-loop structures. However, not terminated TR-aptamers with additional 166 bases after the Terminator Tφ derived from the transfer TR-vector showed a 2-3 time reduction of binding affinity for CD18cyt. Presumably this effect is due to the interference of the additional sequence with the correct folding of the aptamers. These results demonstrate that it is possible to place the aptamers in the context of defined functional sequences which add additional functionalities such as increased stability. Correct termination is also necessary as the functional nucleic acids exhibit reduced ability to bind their target when too extensive additional sequences are present within the molecule.

### (d) Cytoplasmic Expression of the functional nucleic acids

Cytoplasmic RNA expression relies on coinfection of cells with two recombinant vaccinia viruses (Fuerst and Moss, J. Mol. Biol. 206 (1989), 333-348). One virus, vaccinia-T7 (vT7), codes for the bacteriophage T7 RNA polymerase; the other, vTR-aptamer, encodes the sequence of the TR-aptamer and is derived from homologous recombination between vaccinia virus and the TR-aptamer vector. Vaccinia expression constructs (vTR-aptamer) were derived via recombination between the TR vector and wild type vaccinia virus (WR strain), high titer virus stocks were generated as described (Asubel et al. Current protocols in molecular biology (1987), Wiley, New York) and double infections with recombinant vaccinia viruses and a vaccinia virus encoding for T7-RNA polymerase (vT7) were carried out as described previously (Kolanus et al., Cell 86 (1996), 233-242). Briefly, 2 x 10⁶ cells were collected by centrifugation, washed with PBS, resuspended in 300 µl RPMI-medium. 100-150 µl of each virus stock (MOI of ∼10 pfu/cell, sonicated for 10 s) was added and incubated for 2 h at 37° C, followed by addition of 4 ml RPMI/10% fetal calf serum and additional incubation for between 1 and 7 h.

The course of RNA-expression after coinfection of a Jurkat E6 cell line with vT7 and vTR-D31 is shown in Figure 2C. Maximal amounts of RNA are reached 7 h post infection as quantified by dot-blot analysis using a DNA hybridisation probe complementary to the 3'-terminus of the aptamer expression cassette. Approximately 4 x 10⁶ Jurkat E6 cells were infected with vaccinia viruses expressing TR-aptamer RNA and T7-RNA polymerase. RNA was isolated as described (Asubel et al. Current protocols in molecular biology (1987), Wiley, New York) and dot blotted onto a Hybond N membrane (NEN Du Pont) according to the manufacturers protocol. Hybridization of virus-expressed RNA was carried out for 1 h by incubation of cellular RNA in pre-hybridization buffer (5 x SSC, 0.1% SDS, 5x Denhardt's reagent, 5% dextrane sulfate, 100 µg/ml tRNA) at 55° C, followed by incubation in the presence of 20 pmoles of radiolabeled probe MicTR1/28.1 (5'-CAAAAAACCCCTCAAG ACCCGTTTAGAG-3') in a total volume of 4.0 ml for 1 h. Dot-blots were then washed with ∼20 ml 2x SSC, 0.1% SDS (2x), 1x SSC, 0.1% SDS (2x), and 0.5x SSC, 0.1% SDS (2x) at 55° C for 30 min, and quantified by phosphor imaging. Analysis of all TR transcripts *in vivo* at this peak revealed comparable aptamer RNA levels of roughly 10⁶ copies per cell, but only in the presence of vT7 (Figure 2D).

### (e) Specificity of the TR-aptamers for endogenous CD18/β2 Integrin

Before investigating the biological effects of TR-aptamer expression it had to be confirmed that the aptamers are also able to specifically bind endogenous LFA-1. This was demonstrated by incubating radiolabelled aptamers transcribed *in vitro* from TR-aptamer vectors with crude cytoplasmic lysate from Jurkat E6 cells and subsequent gel electrophoresis under native conditions. 4·10⁷ Jurkat E6 cells were lysed in 400 ml of lysis buffer (PBS, 0.5% Triton X 100, 0.5 µg/ml aprotinin, 0.5 µg/ml leupeptin, 1 mM PMSF, 1 mM MgCl₂) and incubated at 0° C for 20 min. Nuclei were removed by centrifugation at 3,000 g for 10 min and the supernatant was clarified by centrifugation at 10,000 g for 10 min. To 3 ml of this lysate, 2 mg of purified monoclonal antibody (MHM23, OKT3) or 2 ml ascites fluid (MEM170) were added and adjusted to 1x PBS in a total volume of 7 µl. After incubation at 0° C for 2-3 h, 5 µl of PBS containing 3 mM DTT, 1 mM MgCl₂, 30 U RNasin, 75-120 µM tRNA, 20% glycerol, 7.5 mg BSA were added and incubated at 0° C for 15 min. Following incubation, 30-60 fmol 5'-³²P-labeled TR-aptamer in 3 µl PBS, 1 mM MgCl₂, heated to 95° C for 30 s and cooled to 23° C for 10 min, was added and incubated at 23° C for 30 min. The lysate/aptamer/antibody mix was loaded onto a native 4.5% polyacrylamide gel (acrylamide/bis-acrylamide 60:1) containing 2% glycerol, and electrophoresed at 150 V for 2.5 h in 0.25 x TAE electrophoresis buffer.

As shown in Figure 3 in two representative experiments, this resulted in a pattern of shifted bands which were aptamer-specific. Aptamer TR-D20 shows a band-pattern with significantly reduced electrophoretic mobility (Figure 3A, lanes 2, 4) in comparison with the negative controls of lysate-free RNA (lane 1) and non-binding TR-D42 (lanes 3, 5) in the presence of a 10⁴-fold excess of tRNA as a non-specific competitor. To test whether the shifts were also LFA-1-specific, we performed a supershift analysis with two independent LFA-1-specific antibodies MHM23 and MEM170, directed against the extracellular domain of the β- and α-subunits of LFA-1, respectively, and the antibody OKT3 which recognises the ε-chain of the T-cell antigen receptor as a negative control. Addition of either MHM23 or MEM170 (lanes 6, 7) resulted in a supershifted band. In Figure 3B again both LFA-1-specific antibodies MHM23 and MEM170 (Figure 3B, lanes 3, 5) show the supershifted bands while no supershift was obtained with the non-cognate antibody OKT3 (lane 6). The experiments shown in lanes 4 and 7 establish that the aptamer TR-D20 has no direct affinity to either the specific antibody MHM23 or the non-specific antibody OKT3. When the gel-shift experiment shown in lane 2 was repeated in the presence of 2 mM unlabeled TR-D20 aptamer as a specific competitor, all radiolabeled TR-D20 RNA migrated at the level of unbound RNA (data not shown). Analogous results were obtained with TR-D28 and TR-D31 showing that the aptamers recognise endogenous CD18 in crude cell lysates with remarkable specificity.

### (f) Determination of the binding site of the TR-aptamers on the intracellular domain of CD18

To define the binding site of the aptamers on the CD18cyt, gel-shift experiments were performed with three synthetic CD18cyt peptide fragments (Figure 5). Gel shifts employing synthetic biotinylated peptides were performed as follows: 5 µl of 3 nM radiolabeled aptamer RNA in PBS pH 7.4 was heated to 95° C for 30s and renatured for 10 min at 23° C. Biotinylated peptide was incubated with 2 mg/ml streptavidine in 20 mM Tris-HCl pH 7.4 in a total volume of 6 µl. To this solution a pre-mix was added to adjust the concentrations in the final 20 µl reaction volume to 1 x PBS, 1 mM DTT, 1 mM MgCl₂, 7.5% glycerol, 2.5 mg/ml BSA, 40 µM tRNA, 2U/ml RNasin, and pre-incubated for 20 min at 23° C. The aptamer solution (5 µl) was added, incubated for 20 min at 23° C, and electrophoresed on a native 6% polyacrylamide gel as described above.

No binding to the carboxy terminal half of CD18cyt (peptide A23) was observed with any of the aptamers, but TR-D20, TR-D28, and, more weakly, TR-D31 all bound to both the N-terminal peptide B16 and the middle portion C17. B16 and C17 overlap by 8 amino acids including a cluster of three basic arginine residues. That this highly positively charged cluster was selected by the aptamers as a binding site on CD18cyt is not surprising because an aptamer is a highly negatively charged molecule. As expected, the negative control aptamer TR-D42 exhibited no binding to any of the three fragments used confirming that the peptide-fragment recognition is aptamer-specific.

### (g) Effects of the intracellular TR-aptamers on the integrin mediated cellular adhesion to intercellular adhesion molecule 1 (ICAM1)

To test whether the cytoplasmic expression of CD18-specific aptamers has an effect on the biological activity of this integrin adhesion assays with Jurkat E6 cells or with peripheral blood mononuclear cells (PBMC) were performed (Coligan et al., (1994) Current protocols in immunology (John Wiley & Sons, New York) . The cells were infected with vaccinia viruses encoding CD18-specific aptamers or various control sequences, and binding of these cells to an important ligand of LFA-1, the ICAM-1 molecule, was assessed *in vitro* . This system has an additional level of control since it was known that TR-aptamer expression is absolutely dependent upon co-infection of cells with a virus expressing T7 polymerase (Figure 2D). The ICAM-1-Rg fusion protein was expressed in COS cells, purified from culture supernatants by Protein A-Sepharose, eluted, resuspended in PBS and coated onto plastic dishes as described (Kolanus et al., Cell 86 (1996), 233-242). 2·10⁶ Jurkat or PBM cells were infected with recombinant vaccinia viruses and incubated for 4-8 h at 37°C. After centrifugation, cells were resuspended in RPMI medium and incubated for 5 min at 37°C with or without the addition of 40 ng/ml PMA. Cells were subsequently allowed to adhere to ICAM-1-Rg coated dishes at 37°C for 30 min and the bound fraction was determined with the aid of an ocular reticle.Figure 4A shows that infected Jurkat E6 cells displayed considerable background adhesion to ICAM-1, but this was nonetheless superinducible by phorbol-12-myristate-13-acetate (PMA), a well known promoter of LFA-1-mediated leukocyte adhesion (Lub et al., Immunol. Today 16 (1995), 479-483). Notably, whereas the TR without insert and single infections with wild-type vaccinia virus (wt), vT7, or vTR-D20 had almost no effect, expression of the TR-D20 aptamer in Jurkat cells resulted in an almost complete block of inducible adhesion.

The reproducibility and extension of the method to other systems was tested by performing similar studies with human peripheral blood mononuclear cells (PBMC). PBMC, which are normally difficult to transfect, proved to be superb targets for transient gene expression mediated by recombinant vaccinia viruses. A flow cytometric analysis of vaccinia expressed control proteins, sub-domain derivatives of cytohesin-1, clearly shows that Ig-fusion proteins with the cytohesin-1 plextrin homology (PH) domain (cIg-PH) and the PH+c-domains (cIg-PH+c), or the isolated Ig portion itself (cIg) were all expressed at equally high levels in human PBMC (Figure 4B). These controls, chosen because the cytoplasmic cytohesin-1 has previously been shown to be an important regulator of β2 integrin adhesive function (Kolanus et al., Cell 86 (1996), 233-242; Nagel et al., J. Biol. Chem. 273 (1998), 14853-14861; Nagel et al., Mol. Biol. Cell 9 (1998), 1981-1994), and the aptamer viruses were tested in adhesion assays with these cells as described above. The cytohesin-1 PH+c-domains (cIg-PH+c) had a strongly suppressive effect on PMA-inducible adhesion whereas both the isolated PH domain (cIg-PH) which shows considerably reduced phospholopid-binding and membrane association (Nagel et al., Mol. Biol. Cell 9 (1998), 1981-1994), or the contol protein had no effect (Figure 4C). These results are in accordance with published data obtained with Jurkat cells (Nagel et al., J. Biol. Chem. 273 (1998), 14853-14861). Most importantly, Figure 4C also shows that TR-D20 aptamer mediated reduction of PMA-inducible adhesion in PBMC is similar to that observed with cIg-PH+c, whereas the negative control sequence TR-D42 had no effect on β2 integrin-mediated adhesion to ICAM-1. Taken together, these results indicate that the adhesive function of LFA-1 was specifically reduced in Jurkat E6- and PBM cells by the cytoplasmic expression of an aptamer selected *in vitro* to bind the cytoplasmic portion of the receptor (Figure 4A,C) (Lub et al., Immunol. Today 16 (1995), 479-483).

### Example 2: Functional target identification of intracellular components involved in the induction of the IL-2 Promotor

Example 2 demonstrates a positive selection scheme incorporating negative selection proofreading to isolate intracellular targets which participate in signal transduction events that lead to the induction of genes under the control of the interleukin 2 promotor (IL-2 P) in T cells. After stimulation of the T-cell receptor (TCR), genes are induced by a signal transduction cascade under the control of the interleukin-2 promotor. Participating in the signal transduction cascade are, among others, proteins such as protein kinase C, calcineurin, NFκB etc. (see: Abbas et al. (Eds.), Cellular and molecular immunology 2nd ed. (1994), p. 162, W.B. Saunders Company). The block of calcineurin by immuno-suppressing pharmaceuticals such as cyclosporin A, FK 506 can, for example, turn off the induction of the promotor and thus the production of cellular cytokines. This is used in the clinic for preventing rejection of organ transplants. The IL-2 promotor is an excellent model system with calcineurin as an example for the clinically relevant functional targets or key-molecules that should be identified.

### a) Phenotypic selection

### (1) Construction of the IL-2-Promotor reporter cell line

A reporter-gene that encodes for a toxin (Herpes simplex virus thymidine kinase, HSV TK) (Fife et al., Gene Ther. 5 (1998), 614-620) under the control of the Il-2 promotor is cloned in a vector containing a selectable marker (aminoglycoside phosphotransferase, neo) (Southern and Berg, J. Mol. Appl. Genet. 1 (1982), 327-341) that allows the selection of cells with the genes of interest inserted in their chromosomes by detoxification of aminoglycosid antibiotics (neomycin, G418) ((Fig.1, plasmid pP1). The IL-2 promotor will be induced when introduced into T cell lines upon stimulation with phorbolesters or via the T cell receptor (TCR) and the herpes simplex virus thymidine kinase (HSV-TK)will be expressed. Addition of ganciclovir results in the formation of toxic compounds (e.g. Kanai et al., Cancer Res. 56 (1996), 1341-5) and subsequent cell death.

After transfection of a T cell line (Jurkat E6 cells ) and culturing in medium (RPMI, 10% FCS, Gentamycin 50 µg/ml) containing 0.1-1.5 mg/ml neomycin (depending on the minimal lethal concentration for the cell line used) and isolation of single stable transfectants the cells were analyzed for the presence and expression of the repotergene via standard methods (e.g. Northern Hybridisation, Western blotting).

### (2) Construction of the RNA expression vectors

A ssDNA library is synthesized , e.g. via standard solid phase synthesis, where the region of random sequence is flanked by fixed sequences that serve as primer annealing sites and contain endonuclease restriction sites for cloning in the expression vectors.dsDNA for example is enzymatically synthesized using PCR or Klenow protocols. This corresponds to the standard protocols for generating nucleic acid libraries for *in vitro* selection experiments (see e.g. Klug et al., Proc.Natl.Acad.Sci 94 (1997), 6676-81; Abelson, Methods in Enzymology 267 (1996), pp 275-81, Academic Press, San Diego)

The library of random sequence genes was cloned under the control of a RNA polymerase III promotor (Pol III P) which allows high level RNA expression (Fig.6, plasmid pP2). Additionally the library is flanked by conserved sequences that are able to form a stem similar to the adenoviral VAI RNA terminal stem (vai random vai)(Furtado et al., J. Virol. 63 (1989), 3423-3434). The resulting RNA transcripts therefor consist of the sequence : 5'-GGGCACUCUUCCGUG-N40-AACGGGGGAGCGCUCCUUUU-3`, whereby N40 represents fourty contigous random positions with equal stochiometry of all four bases (A,C,G,U). It was previously shown that those constructs are exported very efficiently and from the nucleus to the cytoplasm of eukaryotic cells (Bertrand et al., Program & Abstracts RNA `98: The third annual meeting of the RNA Society 26.-31.5.1998, University of Winsconsin, Madison). In this context most of the RNA library is exported into the cytoplasm where most of the targets are located which participate in the induction of the IL-2 promotor.

The plasmid (Fig 6, plasmid pP2) additionally contains a selectable marker (hygromycin) (The antibiotic hygromycin and resistence marker genes are commercially available and described, e.g. Invitrogen BV, Groningen, Netherlands) and elements for episomal replication to ensure maintainance of the plasmid (OriP, EBNA1-protein gene) (Yates et al., Nature 313 (1985), 812-815; Chittenden et al., J. Virol. 63 (1989), 3016-3025). This could be necessary if the selection process proceeds for a longer time period. The library plasmid is transfected into the reporter cell line and the time course, maximum RNA library expression and RNA localisation are determined using standard techniques such as Northern blots and in situ hybridisations. In a typical transfection experiment 10⁷ - 10⁸ cells are transfected using electroporation or lipofection. RNA samples prepared from the transfected cells were isolated and analyzed according to the methods described in Example 1 ((d) Cytoplasmic Expression of the functional nucleic acids) and probed with a radiolabeld antisense oligonucleotide (5`-AAAAGGAGCGCTCCCCCGTT-3'). The study revealed expression levels of roughly 5 x 10⁵ RNA copies/cell.

### (3) Selection:

After transfection and expression of the RNA library the reporter cells (stable transfected Jurkat E6 cells) are now selected for the phenotype of inhibited IL-2 promotor induction. The cells are stimulated with phorbol-12-myristate-13-acetate (PMA) (50 ng/ml) (Kolanus et al., Cell 86 (1996), 233-242) and incubated in the presence of ganciclovir (Obaru et al., Hum. Gene Ther. 7 (1996), 2203-2208). Only those cells expressing a functional nucleic acid from the library that inhibits the induction or expression of the HSV-TK gene (see Figure 8 blocking protein X of the schematically drawn signal transductioncascade X, Y, Z) will survive this selection. Other cells will die (Fig. 7). In order to allow the enrichment of cells the stringency of the selection can be varyied if necessary employing different concentrations of ganciclovir, different incubation periods or repeated cycles of stimulation and incubation with the drug. The remaining cells can be harvested and the plasmids carrying the gene for the inhibiting RNA molecule transfected for amplification into *E.coli* using the "hirt supernatant" method. The cells are lysed in 0.4ml 0.6% SDS, 10 mM EDTA, 1'-20'. The viscous mixture is pipetted into a microfuge tube. After addition of 0.1ml 5M NaCl and mixing, the samples are incubated 5 hrs on ice. After spinning 4 min, carefully removing supernatant, phenol extraction, addition of 10 micrograms linear polyacr acids. Second, all functional nucleic acids can be eliminated that just act by inhibiting the toxin and not act on the signal transduction directly.

### (c) Identification of functional cellular targets

### (1) Construction of the vectors for the functional target identification:

The main functional elements of the suitable plasmid are depicted in Figure 9.

### plasmid pF1:

- Promotor elements:
   CMV P: human cytomegalovirus immediate early promotor. Example of a constitutive promotor element that is active in a wide variety of cell types. Depending on cell lines, application, etc. other frequently used promotors or combinations of promoter elements (E.g. SV 40 early promotor, Rous sarcoma virus immediate early promotor etc.) can be used (Spector et al., Cells: A Laboratory Manual (1998), Cold Spring Harbor Laboratory Press, Cold Spring Harbor, New York, 67.2-67.3).
      IL-2 P: IL-2 Promotor
- Episomal replication:
   The Epstein Barr Virus (EBV) origin of replication (OriP) and nuclear antigen (EBNA-1) allow the high copy episomal (extra chromosomal) replication and maintenance in primate and canine cell lines (Yates et al., Nature 313 (1985), 812-815; Chittenden et al., J. Virol. 63 (1989), 3016-3025). This is an optional element but allows the selection to proceed over a longer time period if necessary.
- cDNA library:
   The cDNA library in this example is derived from T cells. The library is used to select for the complementation of the inhibited signal transduction cascade. Overexpression of the factor that is inhibited by the functional nucleic acid should encompass the block in signalling and the IL-2 promotor be induced again in cells harbouring the target gene of interest. Construction and expression of cDNA libraries are well known to a person skilled in the art.
- fNS-hsv thymidine kinase:
   This construct allows the identification of the target gene product through the specificity of the nucleic acid-protein interaction. The gene of the functional nucleic acid (fNS) is cloned in the 5' untranslated leader of the herpes simplex virus thymidine kinase. Werstuck and Green could show that constructs which harbour aptamer sequences in the 5' untranslated leader are very useful for controlling the expression of genes (*Science* 282 (1998), 296-298). Upon binding of the aptamer ligand (functional nucleic acid) the expression of the reporter HSV TK is inhibited presumably through blocking the translation.
   - Selectable marker:
      neo: commonly used resistance gene (aminoglycosid phosphotransferase) to select for cells harbouring the transfected plasmids via detoxification of aminoglycosid antibiotics (Southern and Berg, J. Mol. Appl. Genet. 1 (1982), 327-341).

### Plasmid pF2:

- Promotor elements (:
   See pF1. Additionally RNA Polymerase III (Pol III P) promotor-VAI/fNS/VAI construct. This construct permits as described in the functional selection step the high level transcription and export of the functional nucleic acid in the cytoplasm.
- Episomal replication: See pF1.
- Selectable marker: Resistance against hygromycin can be used to coselect for cells that harbor two plasmids (e.g. additionally a plasmid with the neo resistance)
- mIg: This is a surface marker that allows the affinity selection of transformed cells. In this example the constant CH2 and CH3 domaines of human IgG1 expressed as transmembrane version (mIg) that lead to their presentation on the cell surface. The cells can be isolated for example using immobilized commercially available antibodies (e.g. AffiniPure goat anti-humanIgG, Fcγ fragment, dianova, Hamburg). Fusionproteins with the constant CH2 and CH3 domains of human Ig molecules are a well established.Technique for the expression of proteins (Kolanus et al., Cell 86 (1996), 233-242).

### (2) The selection process:

After transformation of plasmids pF1 and pF2 in a T cell line (e.g.Jurkat E6 cells) the cells are coselected in the presence of neomycin and hygromycin. The Pol III transcripts of the functional nucleic acid (f NS) will block the signaltransduction cascade (schematically Proteins X, Y, Z) by binding to the endogenous protein X. Overexpression of most proteins (schematically protein M) from the cDNA library will have no effect on the blocking of the signal transduction cascade and the Il-2 promotor constructs will not be induced (Fig. 10). The expression of the cognate target (protein X, Fig.11)) for the functional nucleic acid (fNS) from the cDNA library leads to the complementation of the signalling to the IL-2 promotor by bypassing the block of the functional nucleic acid. The reporter genes for mIg and HSV TK are turned on. The mIg is used to isolate those cells where the signal transduction pathway is rescued via affinity isolation. Because the levels of protein X inside the T-cells are held at saturating concentrations using high level expression promotors protein X also binds to the mRNA of HSV TK and prevents its expression by blocking its translation. The cells will survive culture in the presence of ganciclovir. If necessary several rounds of gene induction and selection can be repeated and finally the plasmids with the cDNA genes be isolated for example using the "hirt supernatant" method.

The elegance of this system is its dual read out. The target gene is identified via two different properties. It's gene product has to be able to functionally rescue the signal transduction in a homologous sytem for the affinity selection of the cells via the mIg. Furthermore, the target protein protects the cells from being killed because it specifically binds to the functional nucleic acid (aptamer) RNA cloned into the 5'-UTR of the HSV-TK mRNA which is induced by the target protein itself. This approach makes use of alternate conformations of RNA-aptamers which can be controlled in dependence of the presence or absence of the cognate aptamer ligand. This is common knowledge because detailed structural investigations of many aptamer/ligand complexes by multi-dimensional NMR spectroscopy have established that RNA/ligand complex formation is always accompanied by extensive conformational changes and conformational stabilization of the binding RNA-receptor only in the presence of the cognate ligand (Patel et al., J. Mol. Biol. 272 (1997), 645-664; Patel, Curr. Opin. Chem. Biol. 1 (1997), 32-46; Fan et al., J. Mol. Biol. 258 (1996), 480-500; Dieckmann et al., RNA 2 (1996), 628-640; Yang et al., Science 272 (1996), 1343-1347). Furthermore, Werstuck and Green [Science 282 (1998), 296-298] established that the translation of a gene can be prevented if an aptamer sequence is inserted into the untranslated region of an expression plasmid in the presence of the aptamer's cognate ligand, but proceeds normally in its absence. The need to be functional and specific in two independent systems reduces the danger of obtaining false positive cDNA clones. Clones that activate the IL-2 promotor by deregulation of the signalling (e.g. protein N acting on protein Z, fig. 12) should fail to bind specifically to the fNS in the HSV-TK mRNA and to block its expression. Therefore they will not survive the selection because of the ganciclovir mediated cell killing. Finally the system provides specific modulators that can be easily used in the targetvalidation described herein to elucidate the complex protein networks inside cells.

**Table 1**

| aptamer | K_{d}s (µM) |
|---|---|
| D20 | 1.08 |
| TR D20 | 1.37 |
| TR-D20 RT | 2.25 |
| D28 | 0.55 |
| TR D28 | 0.53 |
| TR D28-RT | 1.82 |
| D31 | 6.14 |
| TR D31 | 3.88 |
| TR D31 RT | >13.7 |
| Table 1: K_{d}s (µM) of CD18cyt binding aptamers to the cytoplasmatic domain of CD18. TR indicates correctly terminated TR-aptamers in the TR-vector expression cassette context. RT : readthrough of not correctly terminated TR-aptamers with additional 166 nucleotides | |

## Claims

1. A method for the identification of an intramer capable of binding to and modifying the function of a functional intracellular target by a mechanism different from an antisense mechanism, comprising:
a) preparing a candidate mixture of nucleic acids;
b) contacting the candidate mixture of nucleic acids with the intracellular target or a part thereof;
c) selecting and isolating nucleic acids having an increased affinity to said target relative to the candidate mixture;
d) reverse transcribing, if the candidate mixture comprises RNAs and amplifying the nucleic acids obtained in step c);
e) optionally repeating steps b) to d);
f) isolating and sequencing the clones (intramers) obtained in step e); and
g) testing whether the expression product of the insert of the clone obtained in step f) binds to and effects the function of the intracellular target in vivo wherein a T7-RNA expression cassette is used as a cytoplasma expression system which comprises a T7 promoter, a stabilising 5' stem-loop and a 3' terminator Tφ with the sequence of said T7-RNA expression cassette with no insert being as follows: 
5'-GTTAAC-GCATGCTAATACGACTCACTATAG
GGAGACCACAACGGTTTCCCGGGCGCAAGTTACTAGT-TGGCCA-
AGATCT-TAATTAATAGCATAACCCCTTGGGGCCTCTAAACGGG
TCTTGAGGGGTTTTTTGCTGTCGAC-GCGGCCGC-3'
 whereby the insert is inserted between the 5' stem-loop and the terminator Tφ via the XmaI and PacI restriction sites.

2. The method of claim 1 further comprising
h) mapping of the binding site of the intramer to said target.

3. A method for identification of a functional intracellular target which is associated with a particular phenotype and the corresponding intramer capable of binding to and modifying the function of said target by a mechanism different from an antisense mechanism, comprising:
a) preparing a candidate mixture of nucleic acids;
b) cloning the candidate mixture of nucleic acids under the control of a suitable promotor in a vector optionally containing a selectable marker wherein the vector is a cytoplasmic expression system comprising a T7-RNA expression cassette wherein said T7-RNA expression cassette comprises a T7 promotor, a stabilising 5' stem-loop and a 3' terminator Tφ wherein the sequence of the T7-RNA expression cassette with no insert being as follows: 
5′-GTTAAC-GCATGCTAATACGACTCACTATAG
GGAGACCACAACGGTTTCCCGGGCGCAAGTTACTAGT-TGGCCA-
AGATCT-TAATTAATAGCATAACCCCTTGGGGCCTCTAAACGGG
TCTTGAGGGGTTTTTTGCTGTCGAC-GCGGCCGC-3′
 whereby the insert is inserted between the 5' stem-loop and the terminator Tφ via the XmaI and PacI restriction sites.
c) introducing the vector obtained in step b) into a reporter cell line allowing positive or negative phenotypic selection;
d) selecting cells with an altered phenotype; and
e) determining the sequence of the nucleic acid inserted in the vector of step b) (intramer) and the compound which it binds to.

4. The method of any one of claims 1 to 3, wherein said candidate mixture of nucleic acids comprises single stranded nucleic acids.

5. The method of claim 4, wherein the single stranded nucleic acid is a RNA.

6. The method of any one of claim 1 to 5, wherein the functional intracellular target is an integrin.

7. The method of any one of claim 3 to 6, wherein the reporter cell line of step c) allows negative selection.

8. The method of claim 7, wherein the reporter cell line contains a vector comprising a selectable marker and a reporter gene encoding a toxin under the control of an inducible promotor and wherein only cells will survive expressing the vector of step b) which expresses a nucleic acid (intramer) inhibiting a compound which is required for the activation of the promotor controlling the toxin gene, and wherein in step d) the surviving cells are selected.

9. The method of claim 8 wherein the toxin gene is HSV-thymidine-kinase.

10. The method of claim 8 or 9 wherein the promotor controlling the toxin gene is a IL-2 promotor.

11. A host cell containing one or more vectors as recited in claims 3 to 5 having the candidate mixture as insertions and the vector of claim 8.

12. A T7-RNA expression cassette comprising a T7-promotor, followed by two stem-loop structures between which a cloning site is provided wherein the sequence of this T7-RNA expression cassette comprises the following sequence:

5'-GTTAAC-GCATGCTAATACGACTCACTATAG
GGAGACCACAACGGTTTCCCGGGCGCAAGTTACTAGT-TGGCCA-
AGATCT-TAATTAATAGCATAACCCCTTGGGGCCTCTAAACGGG
TCTTGAGGGGTTTTTTGCTGTCGAC-GCGGCCGC-3'

13. The T7-RNA expression cassette of claim 12 comprising the following restriction sites: HpaI, SphI, XmaI, SpeI, BAII, BglII, PacI; SaII and NotI, and wherein the cassette further comprises an aptamer-coding DNA inserted between the 5'- and 3'-stem-loop structures via the XmaI and PacI restriction sites.

14. Use of the T7-RNA expression system in any of the methods according to claim 1 to 10.

## Patentansprüche

1. Verfahren für die Identifizierung eines Intramers, das in der Lage ist, an ein funktionales intrazelluläres Zielmolekül zu binden und dieses zu modifizieren durch einen Mechanismus, der verschieden ist von einem Antisense-Mechanismus, umfassend:
a) Herstellen einer Kandidatenmischung von Nukleinsäuren;
b) Kontaktieren der Kandidatenmischung von Nukleinsäuren mit dem intrazellulären Zielmolekül oder einem Teil davon;
c) Auswählen und Isolieren von Nukleinsäuren mit einer verglichen mit der Kandidatenmischung erhöhten Affinität für das Zielmolekül;
d) reverses Transkribieren, sofern die Kandidatenmischung RNAs umfasst, und Amplifizieren der in Schritt c) erhaltenen Schritte;
e) optional Wiederholen der Schritte b) bis d);
f) Isolieren und Sequenzieren von Klonen (Intrameren), die in Schritt e) erhalten werden; und
g) Testen, ob das Expressionsprodukt des Inserts des in Schritt f) erhaltenen Klons in vivo an das intrazelluläre Zielmolekül bindet und dessen Funktion beeinflusst, wobei eine T7-RNA-Expressionskassette als ein zytoplasmatisches Expressionssystem verwendet wird, welches einen T7-Promotor, eine stabilisierende 5' Stamm-Schleife und einen 3' Terminator Tφ umfasst, wobei die Sequenz der T7-RNA-Expressionskassette ohne Insert wie folgt ist: 
5'-GTTAAC-GCATGCTAATACGACTCACTATAG
GGAGACCACAACGGTTTCCCGGGCGCAAGTTACTAGT-TGGCCA-
AGATCT-TAATTAATAGCATAACCCCTTGGGGCCTCTAAACGGG
TCTTGAGGGGTTTTTTGCTGTCGAC-GCGGCCGC-3'
 , wobei das Insert zwischen der 5' Stamm-Schleife und dem Terminator Tφ über die XmaI- und PacI-Restriktionsstellen eingefügt ist.

2. Verfahren nach Anspruch 1, weiter umfassend
h) Kartieren der Bindungsstelle des Intramers auf dem Zielmolekül.

3. Verfahren zum Identifizieren eines funktionalen intrazellulären Zielmoleküls, das mit einem besonderen Phänotyp verbunden ist, und des korrespondierenden Intramers, das in der Lage ist, an das Zielmolekül zu binden und dessen Funktion zu modifizieren durch einen Mechanismus, der verschieden ist von einem Antisense-Mechanismus, umfassend:
a) Herstellen einer Kandidatenmischung von Nukleinsäuren;
b) Klonieren der Kandidatenmischung von Nukleinsäuren unter der Kontrolle eines geeigneten Promotors in einen Vektor, der optional einen selektierbaren Marker enthält, wobei der Vektor ein zytoplasmatisches Expressionssystem ist, das eine T7-RNA-Expressionkassette umfasst, wobei die T7-RNA-Expressionskassette einen T7-Promotor umfasst, eine stabilisierende 5' Stamm-Schleife und einen 3'-Terminator Tφ, wobei die Sequenz der T7-RNA-Expressionskassette ohne Insert wie folgt ist: 
5'-GTTAAC-GCATGCTAATACGACTCACTATAG
GGAGACCACAACGGTTTCCCGGGCGCAAGTTACTAGT-TGGCCA-
AGATCT-TAATTAATAGCATAACCCCTTGGGGCCTCTAAACGGG
TCTTGAGGGGTTTTTTGCTGTCGAC-GCGGCCGC-3'
 wobei das Insert zwischen der 5' Stamm-Schleife und dem Terminator Tφ über die XmaI- und PacI-Restriktionsstellen eingefugt ist;
c) Einführen des in Schritt b) erhaltenen Vektors in eine Reporterzelllinie, die positive oder negative phänotypische Selektion erlaubt;
d) Auswählen von Zellen mit einem geänderten Phänotyp; und
e) Bestimmen der Sequenz der in den Vektor von Schritt b) eingefügten Nukleinsäure (Intramer) und der Verbindung, an die sie bindet.

4. Verfahren nach einem der Ansprüche 1 bis 3, wobei die Kandidatenmischung aus Nukleinsäuren einzelsträngige Nukleinsäuren umfasst.

5. Verfahren nach Anspruch 4, wobei die einzelsträngige Nukleinsäure eine RNA ist.

6. Verfahren nach einem der Ansprüche 1 bis 4, wobei das funktionale intrazelluläre Zielmolekül ein Integrin ist.

7. Verfahren nach einem der Ansprüche 3 bis 6, wobei die Reporterzelllinie von Schritt c) negative Selektion erlaubt.

8. Verfahren nach Anspruch 7, wobei die Reporterzelllinie einen Vektor enthält, der einen selektierbaren Marker und ein Reportergen, das unter der Kontrolle eines induzierbaren Promotors für ein Toxin kodiert, umfasst, und wobei nur Zellen überleben werden, die den Vektor von Schritt b) exprimieren, der eine Nukleinsäure (Intramer) exprimiert, die eine Verbindung inhibiert, die für die Aktivierung des Promotors erforderlich ist, der das Toxingen steuert, und wobei in Schritt d) die überlebenden Zellen selektiert werden.

9. Verfahren nach Anspruch 8, wobei das Toxingen HSV-Thymidinkinase ist.

10. Verfahren nach Anspruch 8 oder 9, wobei der Toxingen-steuernde Promotor ein IL-2-Promotor ist.

11. Wirtszelle, die einen oder mehrere der in den Ansprüchen 3 bis 5 beschriebenen Vektoren mit der Kandidatenmischung als Insertionen und den Vektor von Anspruch 8 enthält.

12. T7-RNA-Expressionskassette umfassend einen T7-Promotor, gefolgt von zwei Stamm-Schleifen-Strukturen zwischen denen einen Klonierungsstelle vorgesehen ist, wobei die Sequenz dieser T7-RNA-Expressionskassette die folgende Sequenz umfasst:

5'-GTTAAC-GCATGCTAATACGACTCACTATAG
GGAGACCACAACGGTTTCCCGGGCGCAAGTTACTAGT-TGGCCA-
AGATCT-TAATTAATAGCATAACCCCTTGGGGCCTCTAAACGGG
TCTTGAGGGGTTTTTTGCTGTCGAC-GCGGCCGC-3'

13. T7-RNA Expressionskassette nach Anspruch 12 umfassend die folgenden Restriktionsstellen: HpaI, SphI, XmaI, SpeI, BalI, BgIII, PacI, SaII und NotI, und wobei die Kassette weiter eine Aptamer-kodierende DNA umfasst, die zwischen den 5'- und 3'-Stamm-Schleifen-Strukturen vermittels der XmaI- und PacI-Restriktionsstellen eingefügt ist.

14. Verwendung des T7-RNA-Expressionssystems in einem Verfahren nach einem der Ansprüche 1 bis 10.

## Revendications

1. Procédé pour l'identification d'un intramère capable de se lier à une cible intracellulaire fonctionnelle et d'en modifier la fonction par un mécanisme différent du mécanisme antisens, comprenant :
a) la préparation d'un mélange candidat d'acides nucléiques ;
b) la mise en contact du mélange candidat d'acides nucléiques avec la cible intracellulaire ou une partie de celle-ci ;
c) la sélection et l'isolation des acides nucléiques ayant une affinité accrue envers ladite cible relativement au mélange candidat ;
d) la transcription inverse, si le mélange candidat comprend des ARN et l'amplification des acides nucléiques obtenus dans l'étape c) ;
e) la répétition facultative des étapes b) à d) ;
f) l'isolation et le séquençage des clones (intramères) obtenus à l'étape e) ; et
g) l'étape consistant à tester si le produit d'expression de l'insert du clone obtenu dans l'étape f) se lie à la cible intracellulaire in vivo et en effectue la fonction, dans laquelle une cassette d'expression de l'ARN-T7 est utilisée comme système d'expression cytoplasmique qui comprend un promoteur T7, une tige-boucle de stabilisation en 5' et un terminateur Tφ en 3', la séquence de ladite cassette d'expression de l'ARN-T7 sans insert étant la suivante : 
5'-GTTAAC-GCATGCTAATACGACTCACTATAG
GGAGACCACAACGGTTTCCCGGCGCAAGTTACTAGT-TGGCCA-
AGATCT-TAATTAATAGCATAACCCCTTGGGGCCTCTAAACGGG
TCTTGAGGGGTTTTTTGCTGTCGAC-GCGGCCGC-3'
 dans lequel l'insert est inséré entre la tige-boucle en 5' et le terminateur Tφ au moyen des sites de restriction XmaI et PacI.

2. Procédé selon la revendication 1 comprenant en outre
h) la cartographie du site de liaison de l'intramère à ladite cible.

3. Procédé pour l'identification d'une cible intracellulaire fonctionnelle qui est associée à un phénotype particulier et de l'intramère correspondant capable de se lier à ladite cible et d'en modifier la fonction par un mécanisme différent d'un mécanisme antisens, comprenant :
a) la préparation d'un mélange candidat d'acides nucléiques ;
b) le clonage du mélange candidat d'acides nucléiques sous le contrôle d'un promoteur approprié dans un vecteur contenant facultativement un marqueur sélectionnable, dans lequel le vecteur est un système d'expression cytoplasmique comprenant une cassette d'expression de l'ARN-T7, dans lequel ladite cassette d'expression de l'ARN-T7 comprend un promoteur T7, une tige-boucle de stabilisation en 5' et un terminateur Tφ en 3', dans lequel la séquence de la cassette d'expression de l'ARN-T7 sans insert est la suivante : 
5'-GTTAAC-GCATGCTAATACGACTCACTATAG
GGAGACCACAACGGTTTCCCGGGCGCAAGTTACTAGT-TGGCCA-
AGATCT-TAATTAATAGCATAACCCCTTGGGGCCTCTAAACGGG
TCTTGAGGGGTTTTTTGCTGTCGAC-GCGGCCGC-3'
 dans lequel l'insert est inséré entre la tige-boucle en 5' et le terminateur Tφ au moyen des sites de restriction XmaI et PacI.
c) l'introduction du vecteur obtenu dans l'étape b) dans une lignée cellulaire rapporteuse permettant une sélection phénotypique positive ou négative ;
d) la sélection des cellules ayant un phénotype modifié ; et
e) la détermination de la séquence de l'acide nucléique inséré dans le vecteur de l'étape b) (intramère) et du composé auquel il se lie.

4. Procédé selon l'une quelconque des revendications 1 à 3, dans lequel ledit mélange candidat d'acides nucléiques comprend des acides nucléiques simple brin.

5. Procédé selon la revendication 4, dans lequel l'acide nucléique simple brin est un ARN.

6. Procédé selon l'une quelconque des revendications 1 à 5, dans lequel la cible intracellulaire fonctionnelle est une intégrine.

7. Procédé selon l'une quelconque des revendications 3 à 6, dans lequel la lignée cellulaire rapporteuse de l'étape c) permet une sélection négative.

8. Procédé selon la revendication 7, dans lequel la lignée cellulaire rapporteuse contient un vecteur comprenant un marqueur sélectionnable et un gène rapporteur codant pour une toxine sous le contrôle d'un promoteur inductible et dans lequel uniquement les cellules survivront en exprimant le vecteur de l'étape b) qui exprime un acide nucléique (intramère) inhibant un composé qui est nécessaire à l'activation du promoteur contrôlant le gène de la toxine, et dans lequel dans l'étape d) les cellules survivantes sont sélectionnées.

9. Procédé selon la revendication 8 dans lequel le gène de la toxine est la HSV thymidine kinase.

10. Procédé selon la revendication 8 ou 9 dans lequel le promoteur contrôlant le gène de la toxine est un promoteur de l'IL-2.

11. Cellule hôte contenant un ou plusieurs vecteurs tels qu'énumérés dans les revendications 3 à 5 ayant le mélange candidat comme insertions et le vecteur de la revendication 8.

12. Cassette d'expression de l'ARN-T7 comprenant un promoteur T7, suivi de deux structures en tige-boucle entre lesquelles un site de clonage est prévu dans laquelle la séquence de cette cassette d'expression de l'ARN-T7 comprend la séquence suivante :

5'-GTTAAC-GCATGCTAATACGACTCACTATAG
GGAGACCACAACGGTTTCCCGGGCGCAAGTTACTAGT-TGGCCA-
AGATCT-TAATTAATAGCATAACCCCTTGGGGCCTCTAAACGGG
TCTTGAGGGGTTTTTTGCTGTCGAC-GCGGCCGC-3'

13. Cassette d'expression de l'ARN-T7 selon la revendication 12 comprenant les sites de restrictions suivants : HpaI, SphI, XmaI, SpeI, BalI, BglII, PacI ; SalI et NotI, et dans laquelle la cassette comprend en outre un ADN codant pour un aptamère inséré entre les structures en tige-boucle en 5' et en 3' au moyen des sites de restriction XmaI et PacI.

14. Utilisation du système d'expression de l'ARN-T7 dans l'un quelconque des procédés selon les revendications 1 à 10.
